# EUROPEAN PATENT APPLICATION

(11) **EP 1 374 855 A1**
(43) Date of publication of application: **02.01.2004**
(21) Application number: 02708717.0
(22) Date of filing: 29.03.2002
(51) Int. Cl.: A61K 9/08, A61K 9/48, A61K 47/12, A61K 47/20, A61K 47/34

(54) **MEDICINAL SOLUTIONS**

(30) Priority: 30.03.2001 JP 2001099578
(71) Applicant: Takeda Chemical Industries, Ltd., Osaka-shi, Osaka 541-0045 (JP)
(72) Inventor: YAMAGATA, Yutaka, Kobe-shi, Hyogo 654-0122 (JP); MATSUMOTO, Yukihiro, Kobe-shi, Hyogo 651-0056 (JP); AKIYAMA, Yohko, Omihachiman-shi, Shiga 523-0898 (JP)
(74) Representative: von Kreisler, Alek, Dipl.-Chem.
(86) International application number: PCT/JP2002/003145
(87) International publication number: WO 2002/078669

(57) **Abstract**

The present invention relates to a pharmaceutical solution containing a physiologically active non-peptide substance, an organic acid and a biocompatible organic solvent, and provides a pharmaceutical solution wherein a physiologically active non-peptide substance is dissolved at a high concentration.

## Description

### Technical Field

The present invention relates to a pharmaceutical solution comprising a physiologically active non-peptide substance, an organic acid and a biocompatible organic solvent.

### Background Art

As a non-aqueous pharmaceutical solution of a physiologically active non-peptide substance, for example, an injection solution wherein testosterone enanthate is dissolved in a solvent consisting of chlorobutanol and sesame oil (Delatestril, BTG Pharmaceuticals, US), and an injection solution wherein haloperidol decanoate is dissolved in a solvent consisting of benzyl alcohol and sesame oil (Haldol, Ortho-MacNeil Pharmaceutical, US) have been known. In addition, JP-A-05-017356 discloses a liquid composition wherein nifedipine is dissolved in a solvent consisting of polyvinylpyrrolidone and polyethylene glycol.

While the non-aqueous pharmaceutical solutions of physiologically active non-peptide substances such as those mentioned above have been considered, a method for dissolving such physiologically active non-peptide substances at higher concentrations in the solvents, in which the physiologically active non-peptide substances have been dissolved, for the purpose of downsizing the preparations to be administered, has not been reported.

### Disclosure of the Invention

The present inventors have conducted intensive studies in an attempt to solve the aforementioned problem, and first produced a pharmaceutical solution comprising a physiologically active non-peptide substance, an organic acid and a biocompatible organic solvent. Surprisingly, they have found that the physiologically active non-peptide substance dissolves more than the solubility thereof in the biocompatible organic solvent, and that it provides a superior effect on the formulation of preparations, such as downsizing of preparation and the like. Based on these findings, they have investigated further and completed the present invention.

Accordingly, the present invention relates to
(1) a pharmaceutical solution comprising a physiologically active non-peptide substance, an organic acid and a biocompatible organic solvent,
(2) the pharmaceutical solution of the above-mentioned (1), wherein the organic acid is one or more kinds selected from a lower fatty acid, an aliphatic hydroxycarboxylic acid and an aromatic organic acid,
(3) the pharmaceutical solution of the above-mentioned (2), wherein the aliphatic hydroxycarboxylic acid is lactic acid,
(4) the pharmaceutical solution of the above-mentioned (2), wherein the aromatic organic acid is an aromatic hydroxycarboxylic acid,
(5) the pharmaceutical solution of the above-mentioned (4), wherein the aromatic hydroxycarboxylic acid is salicylic acid,
(6) the pharmaceutical solution of the above-mentioned (4), wherein the aromatic hydroxycarboxylic acid is 1-hydroxy-2-naphthoic acid or 3-hydroxy-2-naphthoic acid,
(7) the pharmaceutical solution of the above-mentioned (4), wherein the aromatic hydroxycarboxylic acid is pamoic acid,
(8) the pharmaceutical solution of the above-mentioned (4), wherein the aromatic hydroxycarboxylic acid is a mixture of two or more kinds selected from salicylic acid, 1-hydroxy-2-naphthoic acid, 3-hydroxy-2-naphthoic acid and pamoic acid,
(9) the pharmaceutical solution of the above-mentioned (1), wherein the physiologically active non-peptide substance is a basic substance,
(10) the pharmaceutical solution of the above-mentioned (1), wherein the physiologically active non-peptide substance has a molecular weight of not more than about 1,000,
(11) the pharmaceutical solution of the above-mentioned (1), wherein the biocompatible organic solvent is polyethylene glycol or a fatty acid ester thereof, or dimethyl sulfoxide,
(12) the pharmaceutical solution of the above-mentioned (1), which further comprises a hydrophilic polymer,
(13) the pharmaceutical solution of the above-mentioned (1), wherein the content of the physiologically active non-peptide substance is not less than about 5 wt% of the total weight of the solution,
(14) the pharmaceutical solution of the above-mentioned (1), wherein the content of the organic acid is about 1 wt% to about 40 wt% of the total weight of the solution,
(15) the pharmaceutical solution of the above-mentioned (1), wherein the organic acid is contained in a proportion of about 1/20 to about 100 moles per 1 mole of the physiologically active non-peptide substance,
(16) the pharmaceutical solution of the above-mentioned (1), wherein the physiologically active non-peptide substance is contained at a concentration higher than the solubility of the physiologically active non-peptide substance in the biocompatible organic solvent,
(17) the pharmaceutical solution of the above-mentioned (1), which is a preparation for parenteral administration,
(18) the pharmaceutical solution of the above-mentioned (17), which is a preparation for injection,
(19) a preparation for implantation, which comprises the pharmaceutical solution of the above-mentioned (1),
(20) the pharmaceutical solution of the above-mentioned (1), which is a preparation for oral administration,
(21) a capsule comprising the pharmaceutical solution of the above-mentioned (1), and the like.

The "physiologically active non-peptide substance" to be used in the present invention may be a free form or a salt. As such "salt", for example, metal salt, ammonium salt, salt with organic base, salt with inorganic acid, salt with organic acid, salt with basic or acidic amino acid, and the like can be mentioned. Preferable examples of the metal salt include alkali metal salts such as sodium salt, potassium salt and the like; alkaline earth metal salts such as calcium salt, magnesium salt, barium salt and the like; aluminum salts; and the like. Preferable examples of the salt with organic base include salts with trimethylamine, triethylamine, pyridine, picoline, ethanolamine, diethanolamine, triethanolamine, dicyclohexylamine, N,N-dibenzylethylenediamine and the like. Preferable examples of the salt with inorganic acid include salts with hydrochloric acid, hydrobromic acid, nitric acid, sulfuric acid, phosphoric acid and the like. Preferable examples of the salt with organic acid include salts with formic acid, acetic acid, trifluoroacetic acid, fumaric acid, oxalic acid, tartaric acid, maleic acid, citric acid, succinic acid, malic acid, methanesulfonic acid, benzenesulfonic acid, p-toluenesulfonic acid and the like. Preferable examples of the salt with basic amino acid include salts with arginine, lysin, ornithine and the like, and preferable examples of the salt with acidic amino acid include salts with aspartic acid, glutamic acid and the like.

The "physiologically active non-peptide substance" may be any as long as it is pharmacologically useful, and is preferably a synthetic organic compound. In addition, the peptide of said "physiologically active non-peptide substance" is a peptide consisting only of essential amino acids. As said "synthetic organic compound", for example, a compound mainly containing a hydrophilic moiety having tertiary amine and a highly hydrophobic moiety such as acyclic or cyclic alkyl, aromatic group and the like, a salt thereof and the like can be mentioned. Specific examples thereof include basic and cationic amphiphilic drug [(CAD); Pharmacological Reviews, vol. 42, No. 4, pp. 327-354 and the like] and the like.

As a basic substance preferable as the "physiologically active non-peptide substance", a substance having an acid dissociation constant (pKa) of 3 to 8 can be mentioned. The pKa can be measured by, for example, the "potentiometric method" described in Experimental Chemistry Guide Book, 4th Ed., 9 Electricity Magnetism, edited by The Chemical Society of Japan, (Maruzen) (1991), pp. 288-289.

As the "physiologically active non-peptide substance", for example, a substance showing receptor agonism or antagonism, enzyme inhibitory action, carrier promoting or suppressive action, and the like can be mentioned.

The receptor to which the "physiologically active non-peptide substance" shows agonism or antagonism may be present on the cell surface or intracellularly. Such cell surface receptor includes, for example, ion channel-coupled receptor, G protein-coupled receptor and enzyme-linked receptor. The kind of the ligand for the receptor includes small peptide, protein, amino acid, nucleotide, steroid, fatty acid derivative, nitrogen monoxide, carbon monoxide and the like. Examples of the receptor include luteinizing hormone-releasing hormone (LH-RH) receptor, thyrotropin-releasing hormone (TRH) receptor, corticotropin-releasing factor (CRF) receptor, endorphin receptor, substance P receptor, neurotensin receptor, thyroid-stimulating hormone (TSH) receptor, prolactin (PRL) receptor, follicle-stimulating hormone (FSH) receptor, luteinizing hormone (LH) receptor, adrenocorticotropic hormone (ACTH) receptor and the like.

The enzyme on which the "physiologically active non-peptide substance" shows an inhibitory action is, for example, enzyme of coagulation system, fibrinogenolysis type enzyme, digestive enzyme, phosphorylase, metabolic enzyme, antioxidase and the like. As said enzyme, for example, monoamineoxidase (MAO), angiotensin-converting enzyme, HMG-CoA reductase, cholesterol acyltransferase (ACAT), cyclooxygenase (COX), trypsin, α-chymotrypsin, kallikrein, β-galactosidase, elastase, thrombomodulin, thrombin, coagulation factors (factor 1-factor X), protein C, protein S, plasmin, plasminogen activator, urokinase, proteinkinase C, tyrosine kinase, cytochrome p450 (3A4, 1A, 2C, 2D etc.), superoxide dismutase (SOD) and the like can be mentioned. As the enzyme on which said CAD shows an inhibitory action, for example, those derived from human cell, bacteria, phage or virus and the like can be mentioned. CAD showing such inhibitory action is expected to have an antimicrobial or antivirus action. As said enzyme, for example, crosslinking enzyme transpeptidase, penicillin-binding protein (PBP-1A, PBP-1B, PBP-2, PBP-3, PBP-4, PBP-5, PBP-6), neuraminidase, aminopeptidase A, aminopeptidase B, α-amylase, β-lactamase, reverse transcription enzyme inhibitor and the like can be mentioned.

As a carrier which is promoted or suppressed by the "physiologically active non-peptide substance", for example, passive or active ion channel, glucose transporter, peptide transporter, p-glucoprotein and the like can be mentioned. As the carrier, for example, voltage-dependent sodium channel, calcium-dependent sodium channel, potassium-dependent calcium channel, potassium channel, chloride ion channel, proton pump on gastric mucosa (H⁺, K⁺-ATPase), glucose transporters (GLUT1, GLUT2, GLUT3, GLUT4), PEPT1, MDR1, MDR2, MRP, cMOAT, ACT1 and the like can be mentioned.

As the "physiologically active non-peptide substance", any substance may be used without a particular limitation as long as it has the above-mentioned activity. For example, a substance having antipyretic, analgesic or antiphlogistic effect is exemplified by salicylic acid, sulpyrine, diclofenac, indomethacin, atropine, scopolamine, morphine, pethidine, a salt thereof and the like; a substance having an ataractic action is exemplified by diazepam, lorazepam and the like; a substance having an antibacterial action is exemplified by griseofulvin and the like; a substance having an antibiotic action is exemplified by dibekacin, kanendomycin, lividomycin, tobramycin, amikacin, fradiomycin, sisomicin, tetracycline, oxytetracycline, rolitetracycline, doxycycline, ampicillin, piperacillin, ticarcillin, cephalothin, cephaloridine, cefotiam, cefotiam hexetil, cefsulodin, cefmenoxime, cefmetazole, cefazolin, cefotaxime, cefoperazone, ceftizoxime, moxalactam, thienamycin, sulfazecin, aztreonam or a salt thereof and the like; a substance having an antitumor activity is exemplified by fumagillol, mitomycin C, adriamycin, fluorouracil and the like; a substance having an hypolipidemic action is exemplified by clofibrate and the like; a substance having an antitussive and expectoration action is exemplified by ephedrine, methyl ephedrine, noscapine, codeine, dihydrocodeine, cloperastine, protokylol, isoproterenol, salbutamol, terbutaline or a salt thereof and the like; a substance having a muscle relaxant action is exemplified by pridinol, pancuronium and the like; a substance having an antiepileptic action is exemplified by phenytoin, acetazolamide, chlordiazepoxide and the like; a substance having an antiulcer action is exemplified by metoclopramide and the like; a substance having an antidepressant action is exemplified by clomipramine and the like; a substance having an antiallergic action is exemplified by diphenhydramine, tripelennamine, diphenylpyraline, methoxyphenamine and the like; a substance having a cardiotonic action is exemplified by etilefrine and the like; a substance having an arrhythmic treatment effect is exemplified by alprenolol, bufetolol, oxprenolol and the like; a substance having a vasodilating action is exemplified by oxyfedrine, bamethan and the like; a substance having a hypotensive diuretic action is exemplified by pentolinium, mecamylamine, clonidine and the like; a substance having a diabetes treatment effect is exemplified by glybuzole and the like; a substance having an antituberculous action is exemplified by ethambutol, p-aminosalicylic acid and the like; a substance having an antinarcotic action is exemplified by levallorphan, nalorphine, naloxone, a salt thereof, and the like; and a substance having a hormone action is exemplified by estrogen, luteinizing hormones (LH), dexamethasone, hexestrol, betamethasone, triamcinolone, triamcinolone acetonide, fluocinolone acetonide, prednisolone, hydrocortisone and the like. In addition, as fat-soluble vitamins, vitamin A, vitamin D, vitamin E, vitamin K, folic acid (vitamin M) and the like can be mentioned.

As the "physiologically active non-peptide substance" having the properties of CAD, for example, a substance showing receptor antagonism, such as amiodarone, promethazine, propranolol and the like, a substance showing an enzyme inhibitory action such as chloramphenicol, gentamicin and the like, a substance showing antagonism with carrier such as amitriptyline, imipramine, trimiprasine and the like, and the like can be mentioned.

As the "physiologically active non-peptide substance", for example, a gonadotropin releasing hormone (GnRH) agonist or antagonist, more preferably a GnRH antagonist can be mentioned. Such GnRH has an luteinizing hormone-releasing hormone (LH-RH)-like activity.

The "GnRH antagonist" may be any compound as long as it has a GnRH antagonism, and, for example, embodiments of the following [I] (compound (I) or a salt thereof), [II], [III] and [IV], and the like can be mentioned.

### [I]

A compound represented by: 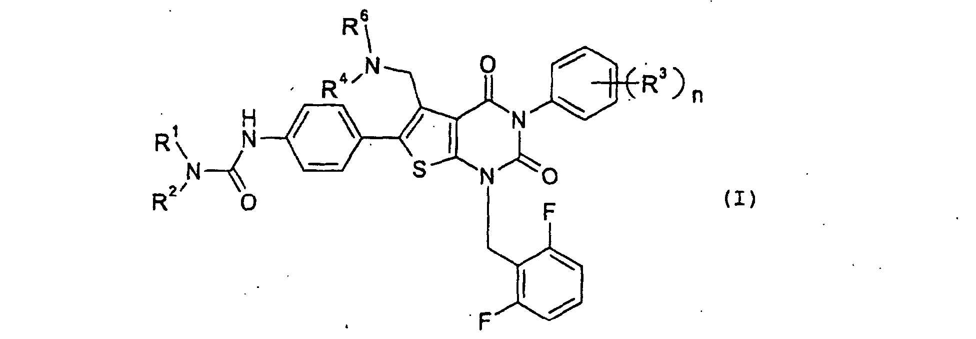 wherein R¹ and R² each represent a hydrogen atom, a hydroxy group, a C₁₋₄ alkoxy group, a C₁₋₄ alkoxy-carbonyl group or a C₁₋₄ alkyl group which may be substituted;
R³ represents a hydrogen atom, a halogen atom, a hydroxy group or a C₁₋₄ alkoxy group which may be substituted; or adjacent two R³ may be taken together to form a C₁₋₄ alkylenedioxy group;
R⁴ represents a hydrogen atom or a C₁₋₄ alkyl group;
R⁶ represents a C₁₋₄ alkyl group which may be substituted or a group of the formula: 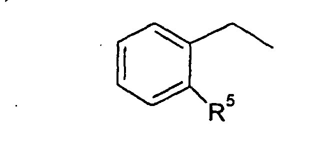 wherein R⁵ represents a hydrogen atom or R⁴ and R⁵ may be taken together to form a heterocycle; and
n represents an integer of 0 to 5; or a salt thereof.

The definition of each substituent in the above formula is given in the following.

The "C₁₋₄ alkoxy group" for R¹ and R² includes, for example, methoxy, ethoxy, propoxy, isopropoxy, butoxy, tert-butoxy and the like. Of these, preferred is a C₁₋₃ alkoxy group. More preferred is methoxy.

The "C₁₋₄ alkoxy-carbonyl group" for R¹ and R² includes, for example, methoxycarbonyl, ethoxycarbonyl, propoxycarbonyl, isopropoxycarbonyl, butoxycarbonyl, tert-butoxycarbonyl and the like. Of these, preferred is a C₁₋₃ alkoxy-carbonyl group. More preferred is methoxycarbonyl.

The "C₁₋₄ alkyl group" of the "C₁₋₄ alkyl group which may be substituted" for R¹ and R² includes, for example, a straight-chain C₁₋₄ alkyl group (e.g., methyl, ethyl, propyl, butyl, etc.), a branched C₃₋₄ alkyl group (e.g., isopropyl, isobutyl, sec-butyl, tert-butyl, etc.), and the like. Of these, preferred is a C₁₋₃ alkyl group. Particularly preferred is ethyl.

The "substituents" of the "C₁₋₄ alkyl group which may be substituted" for R¹ and R² include, for example, (i) hydroxy, (ii) C₁₋₇ acyloxy (e.g., C₁₋₆ alkyl-carbonyloxy such as acetoxy, propionyloxy, etc.), (iii) benzoyloxy, (iv) an amino group which may be substituted by 1 or 2 substituents selected from C₁₋₆ alkoxy-carbonyl (e.g., methoxycarbonyl, ethoxycarbonyl, tert-butoxycarbonyl, etc.), benzyloxycarbonyl, C₁₋₄ acyl (e.g., C₁₋₃ alkyl-carbonyl such as acetyl, propionyl, etc.), C₁₋₄ alkyl (e.g., methyl, ethyl, propyl, butyl, etc.) and C₁₋₃ alkylsulfonyl (e.g., methanesulfonyl etc.), etc. [e.g., amino, dimethylamino, methoxycarbonylamino, ethoxycarbonylamino, tert-butoxycarbonylamino, benzyloxycarbonylamino, acetylamino, methanesulfonylamino, etc.], (v) C₁₋₁₀ alkoxy (e.g., methoxy, ethoxy, propoxy, tert-butoxy, etc.), (vi) C₃₋₇ cycloalkyloxycarbonyloxy-C₁₋₃ alkoxy (e.g., cyclohexyloxycarbonyloxy-1-ethoxy, etc.), (vii) C₁₋₃ alkoxy-C₁₋₃ alkoxy (e.g., methoxymethoxy, methoxyethoxy, etc.), and the like. Of these, preferred is hydroxy.

The "C₁₋₄ alkyl group" of the "C₁₋₄ alkyl group which may be substituted" for R¹ and R² may have, for example, 1 to 5, preferably 1 to 3, substituents as mentioned above at substitutable positions. When the number of substituents is two or more, those substituents may be the same as or different from each other.

Preferably, one of R¹ and R² is a hydrogen atom, and the other is a C₁₋₃ alkoxy group.

The "halogen atom" for R³ includes, for example, fluorine, chlorine, bromine and iodine. Of these, preferred is chlorine.

The "C₁₋₄ alkoxy group" of the "C₁₋₄ alkoxy group which may be substituted" for R³ includes, for example, methoxy, ethoxy, propoxy, isopropoxy, butoxy, tert-butoxy and the like. Of these, preferred is methoxy.

The "substituents" of the "C₁₋₄ alkoxy group which may be substituted" for R³ are the same as those mentioned above for the "substituents" of the "C₁₋₄ alkyl group which may be substituted" for R¹ and R². Of these, C₁₋₁₀ alkoxy is preferable, particularly C₁₋₄ alkoxy, such as methoxy, ethoxy, propoxy, tert-butoxy and the like, is preferable.

The "C₁₋₄ alkoxy group" may have, for example, 1 to 5, preferably 1 to 3, substituents as mentioned above at substitutable positions. When the number of substituents is two or more, those substituents may be the same as or different from each other.

The "C₁₋₄ alkylenedioxy group" formed by adjacent two R³ in combination includes, for example, methylenedioxy, ethylenedioxy and the like.

R³ is preferably a hydrogen atom.

The "C₁₋₄ alkyl group" for R⁴ includes, for example, a straight-chain C₁₋₄ alkyl group (e.g., methyl, ethyl, propyl, butyl, etc.), a branched C₃₋₄ alkyl group (e.g., isopropyl, isobutyl, sec-butyl, tert-butyl, etc.), and the like. Of these, preferred is a C₁₋₃ alkyl group. Particularly preferred is methyl.

The "C₁₋₄ alkyl group which may be substituted" for R⁶ includes, for example, those similar to "C₁₋₄ alkyl group which may be substituted" for R¹ and R².

The "heterocycle" formed by R⁴ and R⁵ in combination includes, for example, a 5- or 6-membered nitrogen-containing heterocyclic group. When R⁴ and R⁵ are bonded, examples of the group of the formula: 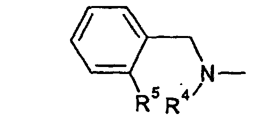 include a group of the formula: 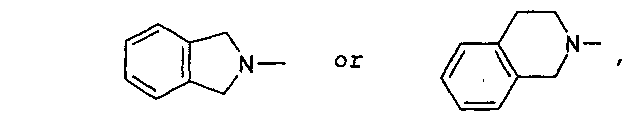 and the like. Of these, preferred is a group of the formula:

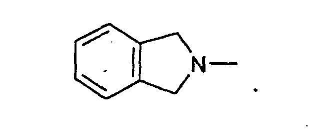

Preferably, R⁶ is a group of the formula: 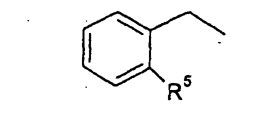 wherein R⁵ is as defined above.

Preferably, R⁴ is a C₁₋₃ alkyl group and R⁵ is a hydrogen atom.

Preferably, n is an integer of 0 to 2.

Preferable examples of compound (I) include a compound or a salt thereof, wherein R¹ is a hydroxy group, a methoxy group or a C₁₋₃ alkyl group; R² is a hydrogen atom or a C₁₋₃ alkyl group; R⁴ is a C₁₋₃ alkyl group; R⁶ is a benzyl group; and n is 0, and the like.

Of these, more preferred are a compound wherein R¹ is a methoxy group; R² is a hydrogen atom; R⁴ is a C₁₋₃ alkyl group; R⁶ is a benzyl group; and n is 0, a salt thereof and the like.

As compound (I), concretely mentioned are
5-(N-benzyl-N-methylaminomethyl)-1-(2,6-difluorobenzyl)-6-[4-(3-methoxyureido)phenyl]-3-phenylthieno[2,3-d]pyrimidine-2,4(1H,3H)-dione,
5-(N-benzyl-N-methylaminomethyl)-1-(2,6-difluorobenzyl)-6-[4-(3-hydroxyureido)phenyl]-3-phenylthieno[2,3-d]pyrimidine-2,4(1H,3H)-dione,
5-(N-benzyl-N-methylaminomethyl)-1-(2,6-difluorobenzyl)-6-[4-(3-methylureido)phenyl]-3-phenylthieno[2,3-d]pyrimidine-2,4(1H,3H)-dione,
5-(N-benzyl-N-methylaminomethyl)-1-(2,6-difluorobenzyl)-6-[4-(3-ethylureido)phenyl]-3-phenylthieno[2,3-d]pyrimidine-2,4(1H,3H)-dione, and salts thereof.

Of these, preferred is 5-(N-benzyl-N-methylaminomethyl)-1-(2,6-difluorobenzyl)-6-[4-(3-methoxyureido)phenyl]-3-phenylthieno[2,3-d]pyrimidine-2,4(1H,3H)-dione or a salt thereof.

Salts of compound (I) are preferably physiologically acceptable acid addition salts. Such salts include, for example, salts with inorganic acids (e.g., hydrochloric acid, hydrobromic acid, nitric acid, sulfuric acid, phosphoric acid, etc.), salts with organic acids (e.g., formic acid, acetic acid, trifluoroacetic acid, fumaric acid, oxalic acid, tartaric acid, maleic acid, citric acid, succinic acid, malic acid, methanesulfonic acid, benzenesulfonic acid, p-toluenesulfonic acid, etc.) , and the like. When compound (I) has an acidic group, it may form a physiologically acceptable salt with an inorganic base (e.g., alkali metals and alkaline earth metals such as sodium, potassium, calcium and magnesium, ammonia etc.) or an organic base (e.g., trimethylamine, triethylamine, pyridine, picoline, ethanolamine, diethanolamine, triethanolamine, dicyclohexylamine, N,N_{'}-dibenzylethylenediamine, etc.).

Compound (I) can be produced by, but not particularly limited to, a known method such as a method disclosed in JP-A-9-169768, WO 96/24597 or a method analogous thereto, or a method disclosed in JP-A-2001-278884, WO 00/56739 or a method analogous thereto. More specifically, the following Production method 1 and Production method 2 can be mentioned. Compounds described in the reaction scheme may form a salt, which is exemplified by those similar to the salts of compound (I) and the like.

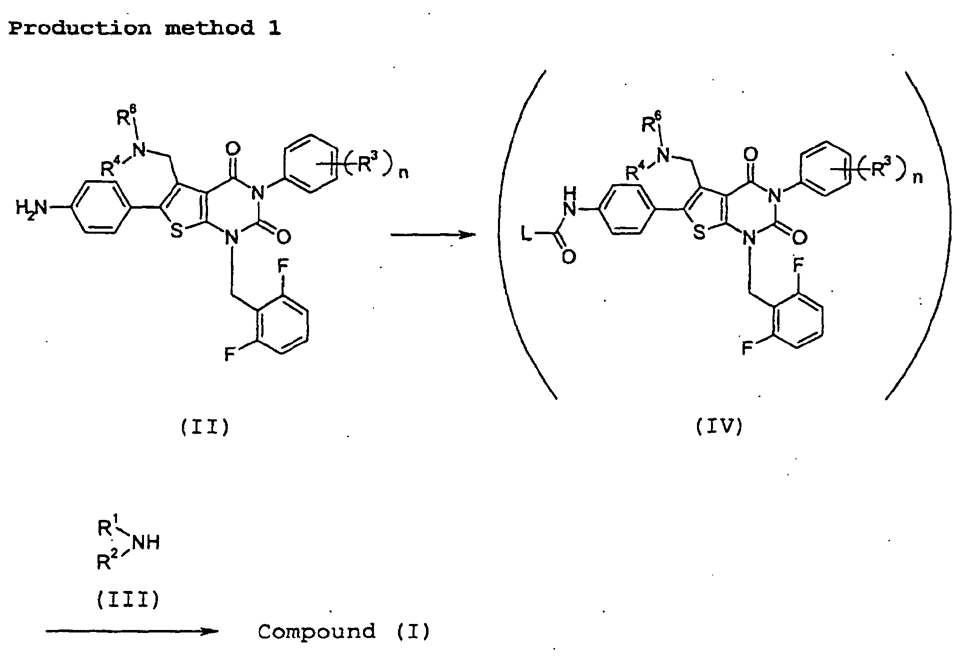

In the above scheme, L represents a leaving group, and other symbols are as defined above.

The "leaving group" for L includes, for example, 1-imidazolyl, a halogen atom, an alkoxy group which may be substituted, and the like. The "alkoxy group which may be substituted" includes, for example, a C₁₋₄ alkoxy group which may be substituted by 1 to 3 halogen atoms such as chlorine, bromine, etc. (e.g., a 2,2,2-trichloroethoxy group, etc.) and the like.

Compound (II), for example, can be produced by the methods disclosed in JP-A-9-169768, WO 96/24597 or analogous methods thereto.

Compound (III) can be obtained by a known method.

Compound (I) can be produced by reacting compound (II) with carbonyldiimidazole (N,N'-carbonyldiimidazole; CDI) or phosgene (inclusive of dimer and trimer) and the like to obtain compound (IV), followed by a reaction with compound (III). The next reaction can be carried out without isolation of compound (IV), or the compound may be isolated and used in the next step.

Compound (IV) can be also produced by reacting compound (II) with a chloroformate compound (e.g., 2,2,2-trichloroethyl chloroformate, 1-chloroethyl chloroformate, etc.) and the like.

In the reaction of compound (II) with carbonyldiimidazole or phosgene, and the like, carbonyldiimidazole or phosgene, and the like is used in an amount of about 1 to 3 moles relative to 1 mole of compound (II).

This reaction is generally carried out in a suitable solvent inert to the reaction.

Examples of the solvent include ethers (e.g., ethyl ether, dioxane, dimethoxyethane, tetrahydrofuran, etc.), aromatic hydrocarbons (e.g., benzene, toluene, etc.), amides (e.g., dimethylformamide, dimethylacetamide, etc.), halogenated hydrocarbons (e.g., chloroform, dichloromethane, etc.), and the like.

The reaction temperature is usually about 0 to about 150°C, preferably room temperature (about 15 to about 25°C).
The reaction time is usually about 1 to about 36 hours.

This reaction is carried out in the presence of a base where necessary.

The "base" is exemplified by inorganic bases such as sodium carbonate, sodium hydrogen carbonate, potassium carbonate, potassium hydrogen carbonate, sodium hydroxide, potassium hydroxide and thallium hydroxide, and organic bases such as triethylamine and pyridine.

The amount of the "base" to be used is about 2 moles to about 20 moles, preferably about 5 moles to about 12 moles, relative to 1 mole of compound (II).

The following reaction with compound (III) can be carried out under the same conditions as those for the reaction of compound (II) with carbonyldiimidazole or phosgene. The amount of compound (III) to be used is about 2 to about 20 moles, preferably about 5 to about 10 moles, relative to 1 mole of compound (II) or compound (IV). The reaction temperature is usually about 0 to about 150°C, preferably room temperature (about 15 to about 25°C). The reaction time is usually about 1 to about 6 hours.

In addition, compound (III) and carbonyldiimidazole or phosgene may be reacted simultaneously with compound (II).

**Production method 2**

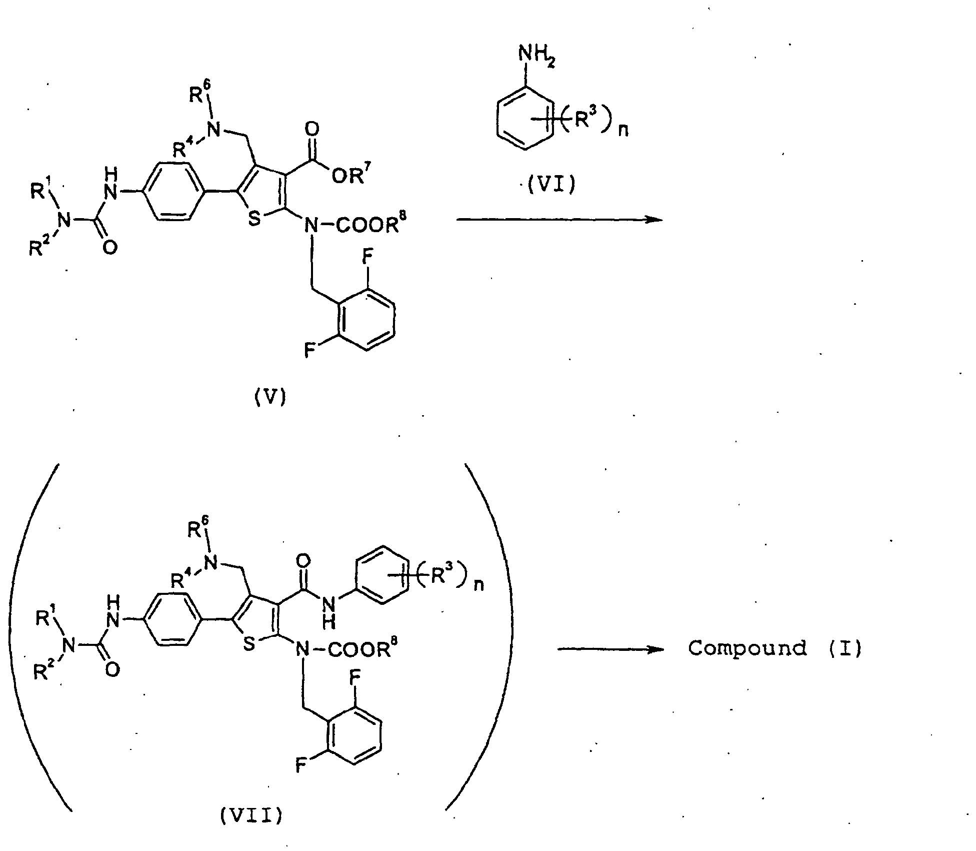

In the above scheme, R⁷ represents a hydrogen atom or an alkyl group, R⁸ represents an alkyl group, and other symbols are as defined above.

The "alkyl group" for R⁷ and R⁸ is exemplified by those similar to the "C₁₋₄ alkyl group" of the "C₁₋₄ alkyl group which may be substituted" for R¹ and R².

Compound (V) can be produced by a known method such as a method comprising reacting p-nitrophenylacetone, a cyanoacetate derivative and sulphur (e.g., Chem. Ber., 99, 94-100(1966) etc.), and subjecting the obtained 2-amino-4-methyl-5-(4-nitrophenyl)thiophene to the methods disclosed in JP-A-9-169768, WO 96/24597 and the like, or methods analogous thereto.
1) When R⁷ is a hydrogen atom, compound (I) is produced by reacting compound (V) with a compound of the formula: 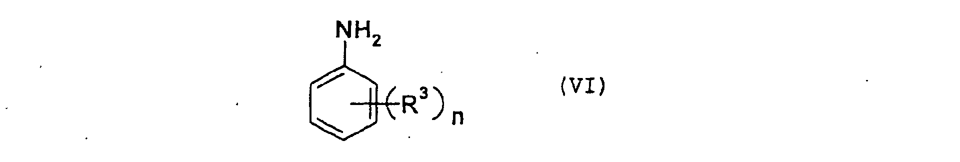 wherein each symbol is as defined above, or a salt thereof [hereinafter sometimes referred to briefly as compound (VI)], in the presence of a condensing agent, to obtain compound (VII), and subjecting the compound to cyclization.
   The "condensing agent" includes, for example, benzotriazol-1-yloxytripyrrolidinophosphonium hexafluorophosphate (PyBOP) and the like.
   The amount of the "condensing agent" to be used is about 1 to about 3 moles relative to 1 mole of compound (V).
   This reaction is generally carried out in a suitable solvent inert to the reaction.
   Examples of the solvent include alcohols (e.g., ethanol, methanol, etc.), aromatic hydrocarbons (e.g., benzene, toluene, etc.), amides (e.g., dimethylformamide, dimethylacetamide, etc.), halogenated hydrocarbons (e.g., chloroform, dichloromethane, etc.) and the like.
   The reaction temperature is usually about 0 to about 150°C, preferably room temperature (about 15 to about 25°C). The reaction time is usually about 1 to about 36 hours.
   The product may be applied to the next reaction in the form of a reaction mixture or as a crude product. It is also possible to isolate the product from the reaction mixture according to a conventional method.
   Compound (VII) can be subjected to cyclization in the presence of a base to give compound (I).
   The "base" is exemplified by inorganic bases such as sodium methoxide, sodium carbonate, sodium hydrogen carbonate, potassium carbonate, potassium hydrogen carbonate, sodium hydroxide, potassium hydroxide and thallium hydroxide, and organic bases such as triethylamine and pyridine.
   The amount of the "base" to be used is about 2 moles to about 20 moles, preferably about 5 moles to about 12 moles, relative to 1 mole of compound (VII).
   This reaction is generally carried out in a suitable solvent inert to the reaction.
   Examples of the solvent include alcohols (e.g., ethanol, methanol, etc.), aromatic hydrocarbons (e.g., benzene, toluene, etc.), amides (e.g., dimethylformamide, dimethylacetamide, etc.), halogenated hydrocarbons (e.g., chloroform, dichloromethane, etc.) and the like.
   The reaction temperature is usually about 0 to about 150°C, preferably room temperature (about 15 to about 25°C). The reaction time is usually about 1 to about 36 hours.
2) When R⁷ is an alkyl group, compound (I) is produced by reacting compound (V) with an activated compound (VI).

The activated compound (VI) can be produced according to a known method and obtained by, for example, reacting an organo-aluminum reagent with compound (VI) in a suitable solvent inert to the reaction.

The "organo-aluminum reagent" includes, for example, trimethyl aluminum, dimethyl aluminum chloride, and the like, a solution including these and the like.

The amount of the "organo-aluminum reagent" to be used is about 1 to about 5 moles, preferably 1 mole, relative to 1 mole of compound (VI).

Examples of the preferable solvent include halogenated hydrocarbons (e.g., chloroform, dichloromethane, etc.).

The reaction temperature is usually about 0 to about 150°C, preferably room temperature (about 15 to about 25°C). The reaction time is usually about 1 to about 6 hours.

The cyclization can be carried out by reacting compound (V) with an activated compound (VI) to obtain compound (I).

The amount of the "compound (V)" to be used is preferably about 1/5 volume of a mixture of compound (VI) and the organo-aluminum reagent.

This reaction is generally carried out in a suitable solvent inert to the reaction.

Such solvent is preferable one used for the reaction to obtain an activated compound (VI).

The reaction temperature is usually about 0 to about 150°C, preferably room temperature (about 15 to about 25°C). The reaction time is usually about 1 to about 48 hours.

Compound (I) may be isolated and purified by a known separation method, such as recrystallization, distillation chromatography, and the like.

When compound (I) is obtained in a free form, it can be converted to an objective salt by a known method or a method analogous thereto. When compound (I) is obtained in a salt form, it can be converted to a free form or an objective different salt by a known method or a method analogous thereto. Compound (I) may be a hydrate or a non-hydrate. The hydrate is exemplified by monohydrate, sesquihydrate, dihydrate and the like. When compound (I) is obtained as a mixture of optically active substances, it can be resolved into the objective (R)-and (S)-forms by the known optical resolution techniques. Compound (I) may be labeled with an isotope (e.g., ³H, ¹⁴C, ³⁵S and the like) and the like.

### [II]

A compound represented by the formula (VIII): 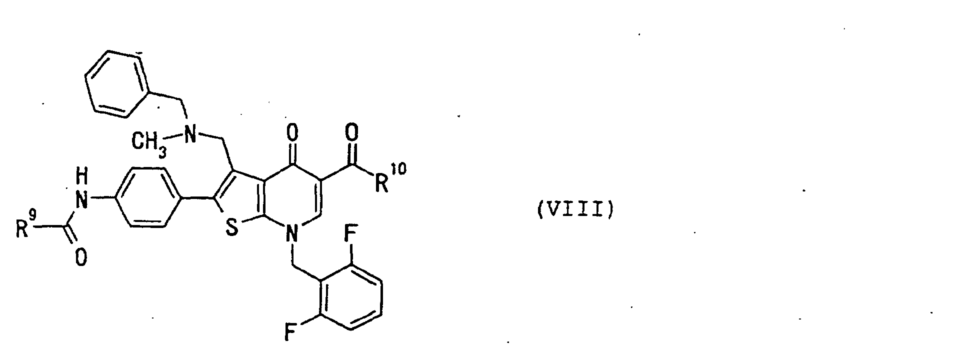 wherein R⁹ represents a C₁₋₇ alkyl group which may be substituted, a C₃₋₇ cycloalkyl group which may be substituted, a C₁₋₆ alkoxyamino group which may be substituted or a hydroxyamino group which may be substituted; and
R¹⁰ represents a C₁₋₇ alkyl group which may be substituted or a phenyl group which may be substituted;
when R⁹ is an unsubstituted C₁₋₇ alkyl group, then R¹⁰ is a substituted C₁₋₇ alkyl group or substituted phenyl group,
or a salt thereof [hereinafter sometimes to be referred to briefly as compound (VIII)].

The definition of each substituent in the above formula is given in the following.

The "C₁₋₇ alkyl group" of the "C₁₋₇ alkyl group which may be substituted" for R⁹ includes, for example, a straight-chain C₁₋₇ alkyl group (e.g. methyl, ethyl, propyl, butyl, pentyl, hexyl, heptyl, etc.); a branched C₃₋₇ alkyl group (e.g., isopropyl, isobutyl, sec-butyl, tert-butyl, isopentyl, neopentyl, etc.) and the like. Of these, preferred is a branched C₃₋₇ alkyl group. Particularly preferred is isopropyl.

The "substituents" of the "C₁₋₇ alkyl group which may be substituted" for R⁹ include, for example, (i) hydroxy, (ii) C₁₋₇ acyloxy (e.g., C₁₋₆ alkyl-carbonyloxy such as acetoxy, propionyloxy, etc.; benzoyloxy etc.), (iii) amino which may be substituted by 1 or 2 substituents selected from C₁₋₆ alkoxy-carbonyl (e.g., methoxycarbonyl, ethoxycarbonyl, tert-butoxycarbonyl, etc.), benzyloxycarbonyl, C₁₋₃ acyl (e.g., C₁₋₂ alkyl-carbonyl such as acetyl, propionyl, etc.), C₁₋₃ alkylsulfonyl (e.g., methanesulfonyl etc.) and C₁₋₃ alkyl (e.g., methyl, ethyl, etc.), and the like, which is exemplified by amino, methoxycarbonylamino, ethoxycarbonylamino, tert-butoxycarbonylamino, benzyloxycarbonylamino, acetylamino, methanesulfonylamino, methylamino, dimethylamino and the like, (iv) C₁₋₁₀ (preferably C₁₋₄) alkoxy which may be substituted by 1 to 3 substituents selected from C₃₋₇ cycloalkyloxycarbonyloxy (e.g., cyclohexyloxycarbonyloxy, etc.) and C₁₋₃ alkoxy (e.g., methoxy, ethoxy, etc.), which is exemplified by methoxy, ethoxy, propoxy, tert-butoxy, cyclohexyloxycarbonyloxy-1-ethoxy, methoxymethoxy, ethoxymethoxy and the like, (v) C₁₋₆ alkoxy-carbonyl (e.g., methoxycarbonyl, ethoxycarbonyl, propoxycarbonyl, etc.), and the like. Of these, preferred is hydroxy.

The "C₁₋₇ alkyl group" may have, for example, 1 to 5, preferably 1 to 3, substituents as mentioned above at substitutable position(s). When the number of substituents is two or more, those substituents may be the same as or different from each other.

The "C₃₋₇ cycloalkyl group" of the "C₃₋₇ cycloalkyl group which may be substituted" for R⁹ includes, for example, cyclopropyl, cyclobutyl, cyclopentyl, cyclohexyl, cycloheptyl, and the like. Of these, preferred is cyclopropyl.

The "substituents" of the "C₃₋₇ cycloalkyl group which may be substituted" for R⁹ are the same as those mentioned above for the "substituents" of the "C₁₋₇ alkyl group which may be substituted" for R⁹. The number of substituents is 1 to 3. When the number of substituents is two or more, those substituents may be the same as or different from each other.

The "C₁₋₆ alkoxyamino group" of the "C₁₋₆ alkoxyamino group which may be substituted" for R⁹ includes, for example, a mono- or di-C₁₋₆ alkoxyamino group (e.g., methoxyamino, ethoxyamino, dimethoxyamino, diethoxyamino, ethoxymethoxyamino, etc.) and the like. Of these, preferred is a mono-C₁₋₃ alkoxyamino group (e.g., methoxyamino, etc.).

As the "substituents" of the "C₁₋₆ alkoxyamino group which may be substituted" for R⁹, for example, the same number of those similar to the "substituents" of the above-mentioned "C₁₋₇ alkyl group which may be substituted" for R⁹ can be mentioned. When the number of substituents is two or more, those substituents may be the same as or different from each other. The "C₁₋₆ alkoxy group" or the "amino group" of the C₁₋₆ alkoxyamino group may be substituted by the above "substituents".

Such "C₁₋₆ alkoxyamino group which may be substituted" is exemplified by methoxyamino, ethoxyamino, dimethoxyamino, diethoxyamino, ethoxymethoxyamino and the like.

The "substituents" of the "hydroxyamino group which may be substituted" for R⁹ may substitute on the "hydroxy group" of the hydroxyamino group or on the "amino group" of the hydroxyamino group. Such "substituents" on the "hydroxy group" include, for example, (i) a C₁₋₇ acyl group (e.g., C₁₋₆ alkyl-carbonyl such as acetyl, propionyl etc.; benzoyl, etc.), (ii) an amino group which may be substituted by 1 or 2 substituents selected from C₁₋₆ alkoxy-carbonyl (e.g., methoxycarbonyl, ethoxycarbonyl, tert-butoxycarbonyl, etc.), benzyloxycarbonyl, C₁₋₃ acyl (e.g., C₁₋₂ alkyl-carbonyl such as acetyl, propionyl, etc. ), C₁₋₃ alkylsulfonyl (e.g. , methanesulfonyl etc.) and C₁₋₃ alkyl (e.g., methyl, ethyl, etc.) and the like, which is exemplified by amino, methoxycarbonylamino, ethoxycarbonylamino, tert-butoxycarbonylamino, benzyloxycarbonylamino, acetylamino, methanesulfonylamino, nethylamino, dimethylamino and the like, (iii) a C₁₋₁₀ (preferably C₁₋₄) alkyl group which may be substituted by 1 to 3 substituents selected from C₃₋₇ cycloalkyloxycarbonyloxy (e.g., cyclohexyloxycarbonyloxy, etc.) and C₁₋₃ alkoxy (e.g., methoxy, ethoxy, etc.), which is exemplified by methyl, ethyl, propyl, tert-butyl, cyclohexyloxycarbonyloXy-1-ethyl, methoxymethyl, ethoxymethyl and the like, and the like. The "substituents" on the "nitrogen atom of the amino group" include, for example, the groups described in the above (i) to (iii), and the like. Respective substituents for the "hydroxy group" and "amino group" of hydroxyamino group may be the same or different.

Preferable examples of the "hydroxyamino group which may be substituted" include an N-C₁₋₆ alkyl-N-hydroxyamino group (e.g., N-methyl-N-hydroxyamino, N-ethyl-N-hydroxyamino, etc.), N-C₁₋₃ alkyl-N-C₁₋₃ alkoxyamino group (e.g., N-methyl-N-methoxyamino, N-ethyl-N-methoxyamino and the like) and the like. More preferred is an N-C₁₋₃ alkyl-N-hydroxyamino group and the like.

The "C₁₋₇ alkyl group" of the "C₁₋₇ alkyl group which may be substituted" for R¹⁰ includes, for example, a straight-chain or branched C₁₋₇ alkyl group (e.g., methyl, ethyl, propyl, isopropyl, butyl, isobutyl, sec-butyl, tert-butyl, pentyl, isopentyl, neopentyl, hexyl, heptyl, etc.) and the like. Of these, preferred is a C₁₋₃ alkyl group (e.g., methyl, ethyl, propyl, isopropyl, and the like). Particularly preferred is isopropyl.

As the "substituents" of the "C₁₋₇ alkyl group which may be substituted" for R¹⁰, for example, the same number of those similar to the "substituents" of the above-mentioned "C₁₋₇ alkyl group which may be substituted" for R⁹ can be mentioned. When the number of substituents is two or more, those substituents may be the same as or different from each other.

The "substituents" of the "phenyl group which may be substituted" for R¹⁰ includes, for example, halogen (e.g., fluorine, chlorine, bromine, iodine, etc.), a C₁₋₃ alkyl group (e.g., methyl, ethyl, propyl, isopropyl, etc.), and a C₁₋₃ alkoxy group (e.g., methoxy, ethoxy, propoxy, isopropoxy, etc.). Of these, preferred is halogen, more preferred is fluorine.

The "phenyl group" may have, for example, 1 to 5, preferably 1 to 3, substituents as mentioned above at substitutable positions and, when the number of substituents is two or more, those substituents may be the same as or different from each other.

R⁹ is preferably a substituted branched C₃₋₇ alkyl group or a substituted C₃₋₇ cycloalkyl group, more preferably a branched C₃₋₇ alkyl group substituted by hydroxy or a C₃₋₇ cycloalkyl group substituted by hydroxy. Of these, preferred is a C₃₋₇ cycloalkyl group substituted by hydroxy. Also, a C₁₋₃ alkyl group which may be substituted by hydroxy, a C₃₋₇ cycloalkyl group which may be substituted by hydroxy, mono-C₁₋₃ alkoxyamino, an N-C₁₋₃ alkyl-N-hydroxyamino group, a hydroxyamino group and the like are preferred. Especially preferable R⁹ is a cyclopropyl group which may be substituted by hydroxy or a methoxyamino group, and the like. Most preferred is a cyclopropyl group substituted by hydroxy.

R¹⁰ is preferably a C₁₋₇ alkyl group which may be substituted. More preferred is a C₁₋₃ alkyl group which may be substituted by hydroxy, and the like. Especially preferred is isopropyl. Phenyl is also preferred.

Preferable examples of compound (VIII) include a compound wherein R⁹ is a C₁₋₃ alkyl group which may be substituted by hydroxy, a C₃₋₇ cycloalkyl group which may be substituted by hydroxy or a mono-C₁₋₃ alkoxyamino group; and R¹⁰ is a C₁₋₃ alkyl group, or a salt thereof, and the like.

More preferred is a compound wherein R⁹ is (1) a C₁₋₃ alkyl group substituted by 1 or 2 hydroxy, (2) a C₃₋₇ cycloalkyl group substituted by hydroxy, or (3) a C₁₋₃ alkoxyamino group; and R¹⁰ is an isopropyl group or a phenyl group, or a salt thereof, and the like.

In addition, a compound wherein R⁹ is (1) a C₁₋₇ alkyl group which may be substituted by 1 or 2 substituents selected from hydroxy, C₁₋₃ alkyl-carbonyloxy, amino, benzyloxycarbonylamino, C₁₋₃ alkoxy, C₁₋₃ alkoxy-C₁₋₃ alkoxy and C₁₋₃ alkoxy-carbonyl, (2) a C₃₋₇ cycloalkyl group which may be substituted by hydroxy or C₁₋₃ alkyl-carbonyloxy, or (3) a C₁₋₃ alkoxyamino group; and
R¹⁰ is (1) an isopropyl group which may be substituted by hydroxy or (2) a phenyl group, and a salt thereof are preferable.

As preferable compound (VIII), concretely mentioned are 3-(N-benzyl-N-methylaminomethyl)-4,7-dihydro-5-isobutyryl-7-(2,6-difluorobenzyl)-2-(4-cyclopropanecarbonylaminophenyl)-4-oxothieno[2,3-b]pyridine,
5-benzoyl-3-(N-benzyl-N-methylaminomethyl)-7-(2,6-difluorobenzyl)-4,7-dihydro-4-oxo-2-[4-(3-hydroxy-2-methylpropionylamino)phenyl]thieno[2,3-b]pyridine, 5-(4-fluorobenzoyl)-3-(N-benzyl-N-methylaminomethyl)-7-(2,6-difluorobenzyl)-4,7-dihydro-4-oxo-2-(4-cyclopropanecarbonylaminophenyl)thieno[2,3-b]pyridine, 3-(N-benzyl-N-methylaminomethyl)-4,7-dihydro-5-isobutyryl-7-(2,6-difluorobenzyl)-2-[4-(3-hydroxy-2-methylpropionylamino)-phenyl]-4-oxothieno[2,3-b]pyridine, 3-(N-benzyl-N-methylaminomethyl)-4,7-dihydro-5-isobutyryl-7-(2,6-difluorobenzyl)-2-(4-N'-methoxyureidophenyl)-4-oxothieno[2,3-b]pyridine,
3-(N-benzyl-N-methylaminomethyl)-4,7-dihydro-5-isobutyryl-7-(2,6-difluorobenzyl)-2-[4-[(1-hydroxycyclopropyl)-carbonylamino]phenyl]-4-oxothieno[2,3-b]pyridine, (R)-4,7-dihydro-2-[4-(3-hydroxy-2-methylpropionylamino)-phenyl]-7-(2,6-difluorobenzyl)-3-(N-benzyl-N-methyl-aminomethyl)-5-isobutyryl-4-oxothieno[2,3-b]pyridine, 4,7-dihydro-2-[4-(2-hydroxy-2-methylpropionylamino)phenyl]-7-(2,6-difluorobenzyl)-3-(N-benzyl-N-methylaminomethyl)-5-isobutyryl-4-oxothieno[2,3-b]pyridine, 4,7-dihydro-2-[4-(3-hydroxy-3-methylbutyrylamino)phenyl]-7-(2,6-difluorobenzyl)-3-(N-benzyl-N-methylaminomethyl)-5-isobutyryl-4-oxothieno[2,3-b]pyridine, (R)-4,7-dihydro-2-[4-(2,3-dihydroxypropionylamino)phenyl]-7-(2,6-difluorobenzyl)-3-(N-benzyl-N-methylaminomethyl)-5-isobutyryl-4-oxothieno[2,3-b]pyridine, 3-(N-benzyl-N-methylaminomethyl)-5-benzoyl-7-(2,6-difluorobenzyl)-4,7-dihydro-2-[4-[(1-hydroxycyclopropyl)-carbonylamino]phenyl]-4-oxothieno[2,3-b]pyridine, salts thereof and the like.

As the salts of compound (VIII), those similar to the salts of compound (I) can be mentioned.

Compound (VIII) may be a hydrate or non-hydrate. As the hydrate, for example, monohydrate, sesquihydrate, dihydrate and the like can be mentioned.

Compound (VIII) may be labeled with an isotope (e.g., ³H, ¹⁴C, ³⁵S and the like) and the like.

Compound (VIII) can be produced by a known method, such as a method described in JP-A-2000-219690, WO00/00493 or a method analogous thereto.

### [III]

A compound represented by the formula (IX): 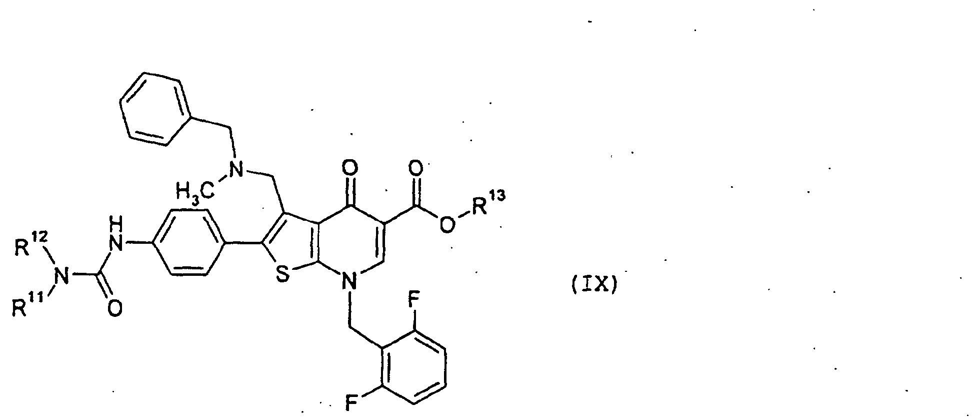 wherein R¹¹ is a hydrogen atom or a C₁₋₃ alkyl group,
R¹² is a hydrogen atom, a hydroxy group or a C₁₋₃ alkoxy group, and
R¹³ is a C₃₋₇ branched alkyl group optionally having substituent(s) or a C₃₋₇ cycloalkyl group optionally having substituent(s) [hereinafter sometimes to be abbreviated as compound (IX)] or a salt thereof.

The definition of each substituent in the formula (IX) is shown in the following.

As "C₁₋₃ alkyl group" for R¹¹, methyl, ethyl, propyl and isopropyl can be mentioned. Of these, methyl and ethyl are preferable. More preferred is methyl.

As "C₁₋₃ alkoxy group" for R¹², methoxy, ethoxy, propoxy and isopropoxy can be mentioned. Of these, methoxy and ethoxy are preferable. More preferred is methoxy.

As the "C₃₋₇ branched alkyl group" of the "C₃₋₇ branched alkyl group optionally having substituent(s)" for R¹³, for example, isopropyl, isobutyl, 1-methylpentyl, 2-methylpentyl, 5-methylhexyl, sec-butyl, tert-butyl, isopentyl, neopentyl, tert-pentyl, isohexyl, 2,4-dimethyl-3-pentyl and the like can be mentioned. Of these, isopropyl, isobutyl, 2,4-dimethyl-3-pentyl and the like are preferable. More preferred is isopropyl.

As the "substituent" of the "C₃₋₇ branched alkyl group optionally having substituent(s)" for R¹³, for example, (i) hydroxy, (ii) C₁₋₇ acyloxy group (e.g., C₁₋₆ alkyl-carbonyloxy such as acetoxy, propionyloxy and the like; benzoyloxy and the like), (iii) C₁₋₁₀ alkoxy group (e.g. , methoxy, ethoxy, propoxy, tert-butoxy and the like) and the like can be mentioned.

The "C₃₋₇ branched alkyl group" may have, for example, 1 to 3 substituents mentioned above at substitutable positions, and when the number of substituents is 2 or more, respective substituents may be the same or different.

As the "C₃₋₇ cycloalkyl group" of the "C₃₋₇ cycloalkyl group optionally having substituent(s)" for R¹³, for example, cyclopropyl, cyclobutyl, cyclopentyl, cyclohexyl, cycloheptyl and the like can be mentioned. Of these, cyclohexyl is preferable.

As the "substituent" of the "C₃₋₇ cycloalkyl group optionally having substituent(s)" for R¹³, the same number of substituents similar to those of the above-mentioned "substituent" can be mentioned. When the number of substituents is 2 or more, the respective substituents may be the same or different.

Examples of preferable compound (IX) include isopropyl {3-(N-benzyl-N-methylaminomethyl)-4,7-dihydro-7-(2,6-difluorobenzyl)-2-[4-(3-methylureido)phenyl]-4-oxothieno[2,3-b]pyridine-5-carboxylic acid ester}, a salt thereof and the like.

As the salt of compound (IX), those similar to the salts of compound (I) can be mentioned.

Compound (IX) can be produced by a known method, such as a method described in WO 95/28405 or a method analogous thereto or a method described in JP-A-2002-030087. In a specific example, a compound represented by the formula (X): 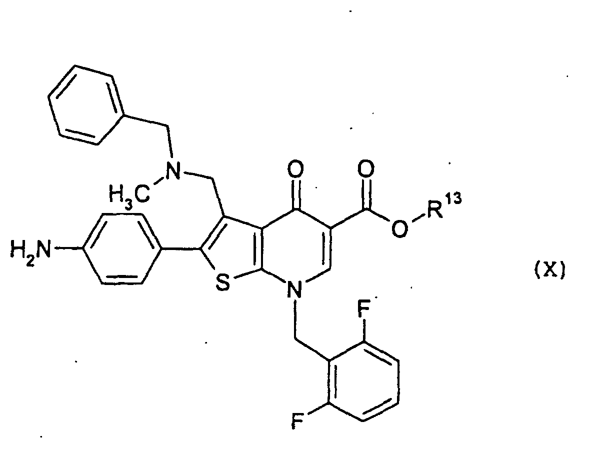 wherein R¹³ is as defined above, or a salt thereof [hereinafter sometimes to be abbreviated as compound (X)] is reacted with carbonyldiimidazole (N,N'-carbonyldiimidazole; CDI) or phosgene (inclusive of dimer and trimer) and the like, and the resulting compound is reacted with compound (XI) represented by the formula (XI): 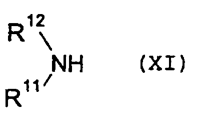 wherein each symbol is as defined above, to give compound (IX).

As the salts of compounds (X) and (XI), for example, those similar to the salts of compound (IX) and the like can be mentioned.

Compound (X) can be produced according to a method described in WO95/28405 or a method analogous thereto. As compound (XI), a commercially available product can be used.

The amount of carbonyldiimidazole or phosgene and the like to be used is each about 1 to about 5 moles per 1 mole of compound (X).

The reaction between compound (X) and carbonyldiimidazole or phosgene and the like is carried out in
a suitable solvent that does not generally exert an adverse influence on the reaction.

As the solvent, for example, ethers (e.g., ethyl ether, dioxane, dimethoxyethane, tetrahydrofuran and the like), aromatic hydrocarbons (e.g., benzene, toluene and the like), amides (e.g., dimethylformamide, dimethylacetamide and the like), halogenated hydrocarbons (e.g., chloroform, dichloromethane and the like) and the like are used.

The reaction temperature is generally about 0 to about 50°C, preferably about 0 to about 25°C. The reaction time is generally about 1 to about 12 hours.

This reaction is carried out in the presence of a base as necessary.

As the "base", for example, an inorganic base such as sodium carbonate, sodium hydrogen carbonate, potassium carbonate, potassium hydrogen carbonate, sodium hydroxide, potassium hydroxide, thallium hydroxide and the like, or an organic base such as triethylamine, pyridine and the like is used.

The amount of the "base" to be used is about 1 to about 5 moles, preferably about 1 to about 3 moles, per 1 mole of compound (X).

The conditions of the reaction subsequently carried out with compound (XI) may be similar to those for the reaction between compound (X) and carbonyldiimidazole or phosgene and the like. The amount of compound (XI) to be used is about 1 to about 10 moles, preferably about 1 to about 5 moles, per 1 mole of compound (X). The reaction temperature is generally about 0 to about 50°C, preferably about 0 to about 25°C. The reaction time is generally about 1 to about 12 hours.

In addition, carbonyldiimidazole or phosgene and compound (XI) may be simultaneously reacted with compound (X).

Compound (IX) can be isolated and purified by a known separation means, such as recrystallization, distillation, chromatography and the like.

When compound (IX) is obtained in a free form, it can be converted to an objective salt by a known method or a method analogous thereto. Alternatively, when it is obtained in a salt, it can be converted to a free form or a different objective salt by a known method or a method analogous thereto.

Compound (IX) may be a hydrate or a non-hydrate. As the hydrate, for example, monohydrate, sesquihydrate and dihydrate and the like can be mentioned.

Compound (IX) may be labeled with an isotope (e.g., ³H, ¹⁴C, ³⁵S and the like) and the like.

### [IV]

A compound represented by the formula (XII): 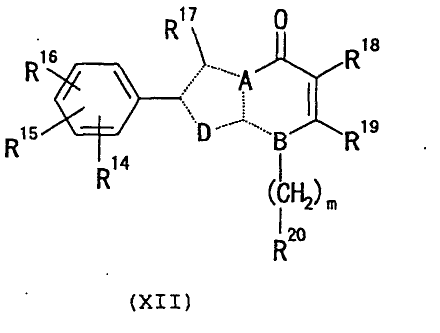 wherein one of A and D represents a nitrogen atom and the other represents a carbon atom, or both represent nitrogen atoms;
B represents a nitrogen atom or a carbon atom;
m represents an integer of 0 to 3;
R¹⁴, R¹⁵ and R¹⁶ are the same or different and each represents (i) a hydrogen atom or (ii) a group bound via a carbon atom, a nitrogen atom, an oxygen atom or a sulfur atom;
R¹⁷ represents a group bound via a carbon atom;
R¹⁸ represents a hydrogen atom, halogen (e.g., fluorine, chlorine, bromine, iodine etc.), or a group bound via a carbon atom or an oxygen atom;
R¹⁹ represents a hydrogen atom or a group bound via a carbon atom;
R²⁰ represents a homocyclic group which may be substituted or a heterocyclic group which may be substituted; and
dotted lines each represent a single bond or a double bond, or a salt thereof (hereinafter sometimes to be abbreviated as compound (XII) or a salt thereof);

The definition of each substituent in the formula (XII) is shown in the following. In the formula, the "group bound via a carbon atom" includes, for example, (1) a hydrocarbon group which may be substituted, (2) an acyl group which may be substituted, (3) a heterocyclic group having a bond in a carbon atom thereof which may be substituted, (4) a carboxyl group which may be esterified or amidated, and (5) a cyano group.

In the formula, the "group bound via a nitrogen atom" includes, for example, (1) a nitro group or (2) a group of the formula: -NR²¹R²² wherein R²¹ represents a hydrogen, a hydrocarbon group which may be substituted, an acyl group which may be substituted, a hydroxy group which may be substituted, a heterocyclic group which may be substituted, or a group of the formula: -S(O)ₜ-R²⁵ wherein t represents an integer of 0 to 2, and R²⁵ represents a hydrogen atom or a C₁₋₁₀ hydrocarbon group which may be substituted; R²² represents a hydrogen, a hydrocarbon group which may be substituted or an acyl group which may be substituted; or R²¹ and R²² may form, taken together with the adjacent nitrogen atom, a cyclic amino group which may be substituted.

In the formula, the "group bound via an oxygen atom" includes, for example, a hydroxy group which may be substituted. The hydroxy group which may be substituted is represented by the formula: -O-R²⁶ wherein R²⁶ represents a hydrogen atom or a C₁₋₁₀ hydrocarbon group which may be substituted, a C₁₋₂₀ acyl group which may be substituted, a C₁₋₂₀ alkylsulfonyl group which may be substituted (e.g., methylsulfonyl, ethylsulfonyl, propylsulfonyl, isopropylsulfonyl, butylsulfonyl, isobutylsulfonyl, sec-butylsulfonyl, tert-butylsulfonyl, pentylsulfonyl, hexylsulfonyl, heptylsulfonyl, octylsulfonyl, nonylsulfonyl, decylsulfonyl, undecylsulfonyl, dodecylsulfonyl, tridecylsulfonyl, tetradecylsulfonyl, pentadecylsulfonyl etc.), a C₆₋₁₄ arylsulfonyl group which may be substituted (e.g., phenylsulfonyl, 1-naphthylsulfonyl, 2-naphthylsulfonyl etc.) or a heterocyclic group which may be substituted.

In the formula, the "group bound via a sulfur atom" includes, for example, a group of the formula: -S(O)ₜ-R²⁷ wherein t represents an integer of 0 to 2, and R²⁷ represents a hydrogen atom or a hydrocarbon group which may be substituted or a heterocyclic group which may be substituted.

The "carboxyl group which may be esterified" includes, for example, a group of the formula: -COO-R³⁴ wherein R³⁴ represents a hydrogen atom or a C₁₋₁₀ hydrocarbon group which may be substituted.

The "carboxyl group which may be amidated" includes, for example, a group of the formula: -CO-NR²⁸R²⁹ wherein R²⁸ represents a hydrogen atom, a hydrocarbon group which may be substituted or an alkoxy group (e.g., C₁₋₆ alkoxy such as methoxy, ethoxy, propoxy, isopropoxy, butoxy, isobutoxy, sec-butoxy, tert-butoxy, pentyloxy, hexyloxy and the like, etc.); R²⁹ represents a hydrogen atom or a hydrocarbon group which may be substituted; or R²⁸ and R²⁹ may form, taken together with the adjacent nitrogen atom, a cyclic amino group which may be substituted. The carboxyl group which may be amidated is preferably exemplified by a group represented by -CONH₂, and mono- or di-C₁₋₁₅ alkylcarbamoyl group, preferably mono- or di-C₁₋₁₀ alkylcarbamoyl group (e.g., methylcarbamoyl, ethylcarbamoyl, hexylcarbamoyl, dimethylcarbamoyl, methylethylcarbamoyl, etc.) and the like.

The "hydrocarbon group" in the "hydrocarbon group which may be substituted" is preferably, for example, a C₁₋₂₀ hydrocarbon group, preferably a C₁₋₁₀ hydrocarbon group. The C₁₋₂₀ hydrocarbon group is exemplified by (1) a C₁₋₁₅ alkyl group (e.g., methyl, ethyl, propyl, isopropyl, butyl, sec-butyl, tert-butyl, pentyl, hexyl, heptyl, octyl, nonyl, decyl, undecyl, dodecyl, tridecyl, tetradecyl, pentadecyl and the like; preferably C₁₋₁₀ alkyl, particularly preferably a C₁₋₆ alkyl group), (2) a C₃₋₁₀ cycloalkyl group (e.g., cyclopropyl, cyclobutyl, cyclopentyl, cyclohexyl, cycloheptyl, cyclooctyl, cyclononyl and the like; preferably a C₃₋₆ cycloalkyl group), (3) a C₂₋₁₀ alkenyl group (e.g. , vinyl, allyl, isopropenyl, 1-butenyl, 2-butenyl, 3-butenyl, butadienyl, 2-methylallyl, hexatrienyl, 3-octenyl, etc.; preferably a C₂₋₆ alkenyl group), (4) a C₂₋₁₀ alkynyl group (e.g., ethynyl, 2-propynyl, butynyl, 3-hexynyl, etc.; preferably a C₂₋₆ alkynyl group), (5) a C₃₋₁₀ cycloalkenyl (e.g., cyclopropenyl, cyclopentenyl, cyclohexenyl, etc.; preferably a C₃₋₆ cycloalkenyl group), (6) a C₆₋₁₄ aryl group (e.g., phenyl, naphthyl, anthryl, phenanthryl, acenaphthyl, etc.; preferably phenyl and naphthyl), (7) a C₇₋₂₀ aralkyl group (e.g., a C₆₋₁₄ aryl-C₁₋₆ alkyl group such as benzyl, phenethyl and benzhydryl, preferably a phenyl-C₁₋₆ alkyl group such as benzyl and phenethyl), and the like.

Such hydrocarbon groups may have 1 to 6, preferably 1 to 5, and more preferably 1 to 3, substituents at any substitutable positions. Such substituents include, for example, (1) halogen (e.g., fluorine, chlorine, bromine, iodine etc.), (2) nitro, (3) nitroso, (4) cyano, (5) hydroxy which may be substituted, such as hydroxy which may be substituted by (i) C₁₋₆ alkyl [the C₁₋₆ alkyl may be substituted by 1 to 3 substituents such as hydroxy, C₁₋₆ alkoxy, C₁₋₃ alkoxy-C₁₋₃ alkoxy, C₁₋₃ alkylthio, hydroxy-C₁₋₃ alkoxy, C₁₋₆ alkyl-carbonyl, carboxy, carbamoyl, C₁₋₆ alkyl-carbamoyl, a 5- to 8-membered heterocyclic group (same as the "5- to 8-membered heterocyclic group containing 1 to 4 hetero atoms selected from oxygen atoms, sulfur atoms, nitrogen atoms etc., in addition to carbon atoms" described below) and halogen (e.g., fluorine, chlorine, bromine, iodine etc.)], (ii) C₁₋₄ acyl (e.g., C₁₋₄ alkanoyl (e.g., formyl, acetyl, propionyl, butyryl, isobutyryl etc.), C₃₋₄ alkenoyl (e.g., vinylcarbonyl, 1-propenylcarbonyl, 2-propenylcarbonyl etc.) , etc.), (iii) C₇₋₂₀ aralkyl (the C₇₋₂₀ aralkyl group is C₆₋₁₄ aryl-C₁₋₆ alkyl and may be substituted by 1 to 3, preferably 1, halogen, C₁₋₃ alkoxy or C₁₋₄ alkyl), (iv) C₆₋₁₄ aryl (the C₆₋₁₄ aryl may be substituted by 1 to 3, preferably 1, halogen (e.g., fluorine, chlorine, bromine, iodine etc.)), (v) C₂₋₆ alkenyl, (vi) C₃₋₇ cycloalkyl, (vii) C₁₋₃ alkoxy-carbonyl, (viii) mono- or di-C₁₋₆ alkylamino, (ix) C₂₋₆ alkenylamino, (x) C₁₋₃ alkoxy-carbonyl, (xi) C₁₋₆ alkyl-carbonyl or (xii) C₃₋₆ cycloalkyloxy-carbonyl, (6) a group of the formula: -S (O) ₜ-R³⁰ wherein t represents an integer of 0 to 2; R³⁰ represents a hydrogen atom or a hydrocarbon group which may be substituted by 1 to 3, preferably 1, substituent (e.g., halogen (e.g., fluorine, chlorine, bromine, iodine etc.), nitro, cyano, hydroxy, oxo, thioxo, carboxy, cyano-C₆₋₁₄ aryl, halogeno-C₆₋₁₄ aryl, etc.) at any substitutable positions; the hydrocarbon group is preferably a C₁₋₂₀ hydrocarbon group, particularly C₁₋₆ alkyl, C₆₋₁₄ aryl or C₇₋₂₀ aralkyl, (7) an amino group which may be substituted, such as a group of the formula: -NR³¹R³² wherein R³¹ and R³² are the same or different and each represents a hydrogen atom, C₁₋₆ alkyl, C₁₋₆ alkylamino-C₁₋₆ alkyl, C₁₋₆ alkoxy, C₂₋₆ alkenyl, C₃₋₇ cycloalkyl, phenyl, phenyl-C₁₋₆ alkyl, C₁₋₆ alkanoyl, C₃₋₆ alkenoyl, C₃₋₇ cycloalkyl-carbonyl, phenyl-C₁₋₆ alkyl-carbonyl, C₁₋₆ alkoxy-carbonyl, phenyl-C₁₋₆ alkoxy-carbonyl or a 5- to 8-membered heterocyclic group (same as the "5- to 8-membered heterocyclic group containing 1 to 4 hetero atoms selected from oxygen atoms, sulfur atoms, nitrogen atoms etc., in addition to carbon atoms" described below) (8) a group of the formula: -CO-R³³ wherein R³³ represents (i) a hydrogen atom, (ii) hydroxy, (iii) C₁₋₁₀ alkyl, (iv) C₁₋₆ alkoxy (this alkoxy may be substituted by C₆₋₁₄ aryl which may be substituted by 1 to 3, preferably 1, substituent such as halogen and nitro, at any substitutable positions), (v) C₃₋₆ cycloalkyl, (vi) C₆₋₁₄ aryl, (vii) C₆₋₁₄ aryloxy, (viii) C₇₋₂₀ aralkyl, (ix) an amino group which may be substituted and represented by the formula: -NR²³R²⁴ wherein R²³ is a hydrogen or a C₁₋₁₀ hydrocarbon group which may be substituted, a C₁₋₂₀ acyl group which may be substituted, a hydroxy group which may be substituted, a heterocyclic group which may be substituted or a group represented by the formula: -S(O)ₜ-R²⁵ wherein t is an integer of 0 to 2, R²⁵ is a hydrogen atom or a C₁₋₁₀ hydrocarbon group which may be substituted or a heterocyclic group which may be substituted; R²⁴ is a hydrogen or a C₁₋₁₀ hydrocarbon group; or R²³ and R²⁴ may form, taken together with the adjacent nitrogen atom, a cyclic amino group which may be substituted, or (x) a 5- to 8-membered heterocyclic group (same as the "5- to 8-membered heterocyclic group containing 1 to 4 hetero atoms selected from oxygen atoms, sulfur atoms, nitrogen atoms etc., in addition to carbon atoms" described below) [e.g., C₁₋₆ alkanoyl, C₃₋₆ alkenoyl, C₁₋₆ alkoxy-carbonyl and the like are preferred], (9) a 5- to 8-membered heterocyclic group containing 1 to 4 hetero atoms selected from nitrogen atoms, oxygen atoms and sulfur atoms, (10) sulfo, (11) C₆₋₁₄ aryl, (12) C₃₋₇ cycloalkyl, (13) C₁₋₆ alkylenedioxy (e.g., methylenedioxy, ethylenedioxy, propylenedioxy, 2,2-dimethylenedioxy, etc.), (14) oxo, (15) thioxo, (16) C₂₋₄ alkynyl, (17) C₃₋₁₀ cycloalkyl, (18) C₂₋₁₀ alkenyl (preferably a C₂₋₆ alkenyl group), (19) C₇₋₂₀ aralkyl (i.e. , C₆₋₁₄ aryl-C₁₋₆ alkyl), (20) amidino, and (21) azide, and the like.

Each group used for the explanation of the "substituent" of the above-mentioned "hydrocarbon group" is exemplified by the following.

As C₁₋₁₀ alkyl, for example, methyl, ethyl, propyl, isopropyl, butyl, isobutyl, sec-butyl, tert-butyl (i.e., C₁₋₄ alkyl), pentyl, hexyl (i.e., C₁₋₆ alkyl), heptyl, octyl, nonyl, decyl and the like can be mentioned.

As C₃₋₁₀ cycloalkyl, for example, cyclopropyl, cyclobutyl, cyclopentyl, cyclohexyl (i.e., C₃₋₆ cycloalkyl), cycloheptyl (i.e., C₃₋₇ cycloalkyl), cyclooctyl, cyclononyl, cyclodecyl and the like can be mentioned.

As C₂₋₁₀ alkenyl, for example, vinyl, allyl, isopropenyl, 1-butenyl, 2-butenyl, 3-butenyl, butadienyl, 2-methylallyl, hexatrienyl (i.e., C₂₋₆ alkenyl), 3-octenyl and the like can be mentioned.

As C₂₋₄ alkynyl, for example, ethynyl, 2-propynyl, butynyl and the like can be mentioned.

As C₁₋₆ alkoxy, for example, methoxy, ethoxy, propoxy, isopropoxy (i.e., C₁₋₃ alkoxy), butoxy, isobutoxy, sec-butoxy, tert-butoxy, pentyloxy, hexyloxy and the like can be mentioned.

As C₁₋₃ alkoxy-C₁₋₃ alkoxy, for example, methoxymethoxy, methoxyethoxy, methoxypropoxy, ethoxymethoxy, ethoxyethoxy, ethoxypropoxy, propoxymethoxy, propoxyethoxy, propoxypropoxy and the like can be mentioned.

As C₁₋₃ alkylthio, for example, methylthio, ethylthio, propylthio, isopropylthio and the like can be mentioned.

As hydroxy-C₁₋₃ alkoxy, for example, hydroxymethoxy, 2-hydroxyethoxy, 3-hydroxypropoxy and the like can be mentioned.

As C₁₋₆ alkyl-carbonyl, for example, acetyl, ethylcarbonyl, propylcarbonyl, butylcarbonyl, tert-butylcarbonyl, pentylcarbonyl, hexylcarbonyl and the like can be mentioned.

As C₃₋₇ cycloalkyl-carbonyl, for example, cyclopropylcarbonyl, cyclobutylcarbonyl, cyclopentylcarbonyl, cyclohexylcarbonyl, cycloheptylcarbonyl and the like can be mentioned.

As C₁₋₆ alkoxy-carbonyl, for example, methoxycarbonyl, ethoxycarbonyl, propoxycarbonyl, isopropoxycarbonyl (i.e. , C₁₋₃ alkoxy-carbonyl), butoxycarbonyl, tert-butoxycarbonyl, pentyloxycarbonyl, hexyloxycarbonyl and the like can be mentioned.

As C₃₋₆ cycloalkyloxy-carbonyl, for example, cyclopropyloxycarbonyl, cyclobutyloxycarbonyl, cyclopentyloxycarbonyl, cyclohexyloxycarbonyl and the like can be mentioned.

As phenyl-C₁₋₆ alkyl-carbonyl, for example, benzylcarbonyl, phenethylcarbonyl and the like can be mentioned.

As phenyl-C₁₋₆ alkoxy-carbonyl, for example, benzyloxycarbonyl, phenethyloxycarbonyl and the like can be mentioned.

As C₁₋₆ alkyl-carbamoyl, for example, methylcarbamoyl, ethylcarbamoyl, propylcarbamoyl, isopropylcarbamoyl, butylcarbamoyl, isobutylcarbamoyl, sec-butylcarbamoyl, tert-butylcarbamoyl, pentylcarbamoyl, hexylcarbamoyl and the like can be mentioned.

As C₁₋₆ alkanoyl, for example, formyl, acetyl, propionyl, butyryl, isobutyryl and the like can be mentioned.

As C₃₋₆ alkenoyl, for example, vinylcarbonyl, 1-propenylcarbonyl, 2-propenylcarbonyl, 1-butenylcarbonyl, 1-pentenylcarbonyl and the like can be mentioned.

As C₆₋₁₄ aryl, for example, phenyl, naphthyl, anthryl, phenanthryl, acenaphthyl and the like can be mentioned.

As cyano C₆₋₁₄ aryl, for example, 2-cyanophenyl, 3-cyanophenyl, 4-cyanophenyl and the like can be mentioned.

As halogeno C₆₋₁₄ aryl, for example, 2-fluorophenyl, 3-fluorophenyl, 4-fluorophenyl, 2-chlorophenyl, 3-chlorophenyl, 4-chlorophenyl, 2-bromophenyl, 3-bromophenyl, 4-bromophenyl, 2,6-difluorophenyl, 2,3-dichlorophenyl, 2,4-dichlorophenyl, 2,5-dichlorophenyl, 2,6-dichlorophenyl and the like can be mentioned.

As C₇₋₂₀ aralkyl, namely, C₆₋₁₄ aryl-C₁₋₆ alkyl, for example, benzyloxy, phenethyloxy and the like can be mentioned.

As C₆₋₁₄ aryloxy, for example, phenoxy, 1-naphthyloxy, 2-naphthyloxy and the like can be mentioned.

As mono- or di-C₁₋₆ alkylamino, for example, methylamino, ethylamino, propylamino, isopropylamino, butylamino, dimethylamino, diethylamino and the like can be mentioned.

As C₂₋₆ alkenylamino, for example, vinylamino, allylamino, isopropenylamino, 1-butenylamino, 2-butenylamino, 3-butenylamino, butadienylamino, 2-methylallylamino and the like can be mentioned.

As C₁₋₆ alkylamino-C₁₋₆ alkyl, for example, methylaminomethyl, ethylaminomethyl, propylaminomethyl, methylaminoethyl, ethylaminoethyl and the like can be mentioned.

As phenyl-C₁₋₆ alkyl, for example, benzyl, phenethyl and the like can be mentioned.

Of the substituents on the hydrocarbon groups having substituent(s), (9) 5- to 8-membered heterocyclic group containing 1 to 4 hetero atoms selected from nitrogen atoms, oxygen atoms and sulfur atoms, (11) C₆₋₁₄ aryl, (12) C₃₋₇ cycloalkyl, (16) C₂₋₄ alkynyl, (17) C₃₋₁₀ cycloalkyl group, (18) C₂₋₁₀ alkenyl group and (19) C₇₋₂₀ aralkyl and the like may further have 1 to 4, preferably 1 to 3, substituents at any substitutable positions of the substituent. Such substituents which may be further contained include, for example, 1 to 3, more preferably 1 or 2, groups selected from (1) hydroxy, (2) amino, (3) mono- or di-C₁₋₄ alkylamino (e.g., methylamino, ethylamino, propylamino, dimethylamino, diethylamino, etc.), (4) C₁₋₄ alkoxy (e.g., methoxy, ethoxy, propoxy, isopropoxy, butoxy, isobutoxy, sec-butoxy, tert-butoxy etc.), (5) halogen (e.g., fluorine, chlorine, bromine, iodine etc.), (6) nitro, (7) C₁₋₆ alkyl (e.g., methyl, ethyl, propyl, isopropyl, butyl, isobutyl, sec-butyl, tert-butyl, pentyl, hexyl etc.) and the like.

When the hydrocarbon group is C₃₋₁₀ cycloalkyl, C₃₋₁₀ cycloalkenyl, C₆₋₁₄ aryl or C₇₋₂₀ aralkyl group, it may be substituted by 1 to 3 substituents such as C₁₋₆ alkyl (e.g., methyl, ethyl, propyl, isopropyl, butyl, isobutyl, sec-butyl, tert-butyl, pentyl, hexyl etc.). The C₁₋₆ alkyl may be further substituted by 1 to 3 substituents such as hydroxy, oxo, C₁₋₆ alkoxy (e.g., methoxy, ethoxy, propoxy, isopropoxy, butoxy, isobutoxy, sec-butoxy, tert-butoxy, pentyloxy, hexyloxy etc.), C₁₋₃ alkylthio (e.g., methylthio, ethylthio, propylthio, isopropylthio etc.), halogen (e.g., fluorine, chlorine, bromine, iodine etc.), carbamoyl and the like.

Such substituted C₁₋₆ alkyl is exemplified by formyl (resulting from methyl via substitution by oxo) , carboxyl (resulting from methyl via substitution by oxo and hydroxy), C₁₋₆ alkoxycarbonyl (resulting from methyl via substitution by oxo and alkoxy) (e.g., C₁₋₆ alkoxycarbonyl such as methoxycarbonyl, ethoxycarbonyl and tert-butoxycarbonyl, etc.), hydroxy C₁₋₆ alkyl (e.g., hydroxymethyl, hydroxyethyl, hydroxybutyl, hydroxypropyl, etc.), and C₁₋₃ alkoxy-C₁₋₆ alkyl (e.g., methoxymethyl, ethoxymethyl, ethoxybutyl, propoxymethyl, propoxyhexyl, etc.), and the like.

Although the number of the above-mentioned substituents ranges from 1 to 6, it is preferably 1 to 5, particularly preferably 1 to 3, and most preferably 1 or 2. The number of substituents that the substituents may have is preferably 1 to 4, particularly preferably 1 to 3, and most preferably 1 or 2.

The acyl group of the above-described acyl group which may be substituted, which has been recited as examples of the group bound via a carbon atom, R²¹, R²², R²³ and R²⁶, includes, for example, a C₁₋₂₀ acyl group such as formyl, C₁₋₆ alkyl-carbonyl (e.g., acetyl, ethylcarbonyl, propylcarbonyl, tert-butylcarbonyl, etc.), C₁₋₆ alkoxy-carbonyl (e.g., methoxycarbonyl, ethoxycarbonyl, tert-butoxycarbonyl, etc.), C₆₋₁₄ aryl-carbonyl (e.g., benzoyl, naphthoyl, etc.), C₆₋₁₄ aryloxy-carbonyl (e.g., phenoxycarbonyl, etc.), C₇₋₂₀ aralkyl-carbonyl (e.g., C₆₋₁₄ aryl-C₁₋₆ alkyl-carbonyl such as benzylcarbonyl, etc.), C₇₋₂₀ aralkyloxy-carbonyl (e.g., C₆₋₁₄ aryl-C₁₋₆ alkoxy-carbonyl such as benzyloxycarbonyl, etc.), C₂₋₄ alkenyl-carbonyl (e.g., 2-propenylcarbonyl, etc.), C₃₋₆ cycloalkyl-carbonyl (e.g., cyclopropylcarbonyl, etc.) tricyclic C₉₋₁₀ bridged hydrocarbon-carbonyl (e.g., adamantylcarbonyl, etc.) and the like.

Substituents of the acyl group which may be substituted are exemplified by the same substituents as those recited as examples of the substituents of the above-described hydrocarbon group which may be substituted.

In the formula, the heterocyclic group or the heterocyclic group in the heterocyclic group which may be substituted includes, for example, 5- to 8-membered heterocyclic groups containing 1 to 4 hetero atoms selected from oxygen atoms, sulfur atoms, nitrogen atoms etc., in addition to carbon atoms, bicyclic or tricyclic condensed heterocyclic groups resulting from condensation of the same or different 2 or 3 of such heterocyclic groups, and bicyclic or tricyclic condensed heterocyclic groups resulting from condensation of such a heterocyclic group, 1 or 2 benzene rings, and the like.

Examples of the heterocyclic group include (1) 5-membered heterocyclic groups containing 1 to 4 hetero atoms selected from oxygen atoms, sulfur atoms, nitrogen atoms etc., in addition to carbon atoms, such as thienyl, furyl, pyrrolyl, pyrrolinyl, oxazolyl, thiazolyl, pyrazolyl, imidazolyl, imidazolinyl, isoxazolyl, isothiazolyl, 1,2,4-oxadiazolyl, 1,3,4-oxadiazolyl, furazanyl, 1,2,4-thiadiazolyl, 1,2,3-thiadiazolyl, 1,2,5-thiadiazolyl, 1,2,3-triazolyl, 1,2,4-triazolyl, triazinyl, triazolidinyl, and 1H- or 2H-tetrazolyl; (2) 6-membered heterocyclic groups containing 1 to 4 hetero atoms selected from oxygen atoms, sulfur atoms, nitrogen atoms etc., in addition to carbon atoms, such as pyridyl, pyrimidinyl, thiomorpholinyl, morpholinyl, triazinyl, pyrrolidinyl, piperidinyl, pyranyl, thiopyranyl, 1,4-oxazinyl, 1,4-thiazinyl, 1,3-thiazinyl, piperazinyl, triazinyl, oxotriazinyl, pyridazinyl and pyrazinyl; (3) bicyclic or tricyclic condensed heterocyclic groups containing 1 to 4 hetero atoms selected from oxygen atoms, sulfur atoms, nitrogen atoms etc., in addition to carbon atoms, such as benzofuryl, benzothiazolyl, benzoxazolyl, tetrazolo[1,5-b]pyridazinyl, triazolo[4,5-b]pyridazinyl, benzimidazolyl, quinolyl, isoquinolyl, cinnolinyl, phthalazinyl, quinazolinyl, quinoxalinyl, indolizinyl, indolyl, quinolizinyl, 1,8-naphthylidinyl, purinyl, pteridinyl, dibenzofuranyl, carbazolyl, acridinyl, phenanthridinyl, chromanyl, benzoxazinyl, phenazinyl, phenothiazinyl, phenoxazinyl, and the like.

Examples of the substituents of the heterocyclic group which may be substituted include (1) C₁₋₆ alkyl (e.g., methyl, ethyl, propyl, isopropyl, butyl, isobutyl, sec-butyl, tert-butyl, pentyl, hexyl etc.), (2) C₂₋₆ alkenyl (e.g., vinyl, allyl, isopropenyl, 1-butenyl, 2-butenyl, 3-butenyl, butadienyl, 2-methylallyl, hexatrienyl etc.), (3) C₂₋₆ alkynyl (e.g., ethynyl, 2-propynyl, butynyl, 3-hexynyl etc.), (4) C₃₋₆ cycloalkyl (e.g., cyclopropyl, cyclobutyl, cyclopentyl, cyclohexyl etc.), (5) C₅₋₇ cycloalkenyl (e.g., cyclopentenyl, cyclohexenyl, cycloheptenyl etc.), (6) C₇₋₁₁ aralkyl (e.g. , C₆₋₁₀ aryl-C₁₋₅ alkyl such as benzyl and phenethyl, etc., preferably benzyl), (7) C₆₋₁₄ aryl (e.g., phenyl, naphthyl, anthryl, phenanthryl, acenaphthyl, anthracenyl, etc., preferably phenyl), (8) C₁₋₆ alkoxy, (9) C₆₋₁₄ aryloxy (e.g., phenoxy, etc.), (10) C₁₋₆ alkanoyl (e.g., formyl, acetyl, propionyl, butyryl, isobutyryl, etc.), (11) C₆₋₁₄ aryl-carbonyl (e.g., benzoyl, etc.), (12) C₁₋₆ alkanoyloxy (e.g., formyloxy, acetyloxy, propionyloxy, butyryloxy, isobutyryloxy, etc.), (13) C₆₋₁₄ aryl-carbonyloxy (e.g., benzoyloxy, etc.), (14) carboxyl, (15) C₁₋₆ alkoxy-carbonyl (e.g., methoxycarbonyl, ethoxycarbonyl, propoxycarbonyl, isopropoxycarbonyl, butoxycarbonyl, isobutoxycarbonyl, tert-butoxycarbonyl, etc.), (16) carbamoyl, (17) N-mono-C₁₋₄ alkylcarbamoyl (e.g., N-methylcarbamoyl, N-ethylcarbamoyl, N-propylcarbamoyl, N-isopropylcarbamoyl, N-butylcarbamoyl, etc.), (18) N,N-di-C₁₋₄ alkylcarbamoyl (e.g., N,N-dimethylcarbamoyl, N,N-diethylcarbamoyl, N,N-dipropylcarbamoyl, N,N-dibutylcarbamoyl, etc.), (19) 3- to 6-membered cyclic aminocarbonyl (e.g., 1-aziridinylcarbonyl, 1-azetidinylcarbonyl, 1-pyrrolidinylcarbonyl, 1-piperidinylcarbonyl, N-methylpiperazinylcarbonyl, morpholinocarbonyl, etc.), (20) halogen (e.g., fluorine, chlorine, bromine, iodine etc.), (21) mono-, di- or tri-halogeno-C₁₋₄ alkyl (e.g., chloromethyl, dichloromethyl, trifluoromethyl, trifluoroethyl, etc.), (22) oxo, (23) amidino, (24) imino, (25) amino, (26) mono- or di-C₁₋₄ alkylamino (e.g., methylamino, ethylamino, propylamino, isopropylamino, butylamino, dimethylamino, diethylamino, dipropylamino, diisopropylamino, dibutylamino, etc.), (27) a 3- to 6-membered cyclic amino group which may contain 1 to 3 hetero atoms selected from oxygen atoms, sulfur atoms, nitrogen atoms etc., in addition to carbon atoms and a nitrogen atom (e.g., aziridinyl, azetidinyl, pyrrolidinyl, pyrrolinyl, pyrrolyl, imidazolyl, pyrazolyl, imidazolidinyl, piperidino, morpholino, dihydropyridyl, N-methylpiperazinyl, N-ethylpiperazinyl, etc.), (28) C₁₋₆ alkanoylamino (e.g., formylamino, acetylamino, trifluoroacetylamino, propionylamino, butyrylamino, isobutyrylamino, etc.), (29) benzamido, (30) carbamoylamino, (31) N-C₁₋₄ alkylcarbamoylamino (e.g., N-methylcarbamoylamino, N-ethylcarbamoylamino, N-propylcarbamoylamino, N-isopropylcarbamoylamino, N-butylcarbamoylamino, etc.), (32) N,N-di-C₁₋₄ alkylcarbamoylamino (e.g., N,N-dimethylcarbamoylamino, N,N-diethylcarbamoylamino, N,N.-dipropylcarbamoylamino, N,N-dibutylcarbamoylamino, etc.), (33) C₁₋₃ alkylenedioxy (e.g., methylenedioxy, ethylenedioxy, etc.), (34) -B (OH)₂, (35) hydroxy; (36) epoxy (-O-), (37) nitro, (38) cyano, (39) mercapto, (40) sulfo, (41) sulfino, (42) phosphono, (43) sulfamoyl, (44) C₁₋₆ alkylsulfamoyl (e.g., N-methylsulfamoyl, N-ethylsulfamoyl, N-propylsulfamoyl, N-isopropylsulfamoyl, N-butylsulfamoyl, etc.), (45) di-C₁₋₆ alkylsulfamoyl (e.g., N,N-dimethylsulfamoyl, N,N-diethylsulfamoyl, N,N-dipropylsulfamoyl, N,N-dibutylsulfamoyl, etc.), (46) C₁₋₆ alkylthio (e.g., methylthio, ethylthio, propylthio, isopropylthio, butylthio, sec-butylthio, tert-butylthio, etc.), (47) phenylthio, (48) C₁₋₆ alkylsulfinyl (e.g., methylsulfinyl, ethylsulfinyl, propylsulfinyl, butylsulfinyl, etc.), (49) phenylsulfinyl, (50) C₁₋₆ alkylsulfonyl (e.g., methylsulfonyl, ethylsulfonyl, propylsulfonyl, butylsulfonyl, etc.), and (51) phenylsulfonyl and the like.

The number of substituents of the heterocyclic group which may be substituted is 1 to 6, preferably 1 to 3, and more preferably 1 or 2.

The heterocyclic group of the heterocyclic group having a bond in a carbon atom thereof which may be substituted is exemplified by 5- to 8-membered heterocyclic groups containing 1 to 4 hetero atoms selected from oxygen atoms, sulfur atoms, nitrogen atoms etc., in addition to carbon atoms, bicyclic or tricyclic condensed heterocyclic groups resulting from condensation of the same or different 2 or 3 of such heterocyclic groups, and bicyclic or tricyclic condensed heterocyclic groups resulting from condensation of such a heterocyclic group and 1 or 2 benzene rings, etc., which heterocyclic groups having a bond in a constituent carbon atom thereof.

Examples of the heterocyclic group having a bond in a carbon atom thereof include (1) a 5-membered heterocyclic group containing 1 to 4 hetero atoms selected from oxygen atoms, sulfur atoms, nitrogen atom(s) etc., in addition to carbon atoms, and having a bond in a carbon atom thereof, such as thienyl (e.g., 2- or 3-thienyl, etc.), furyl (e.g., 2- or 3-furyl, etc.), pyrrolyl (e.g., 2- or 3-pyrrolyl, etc.), oxazolyl (e.g., 2-, 4- or 5-oxazolyl, etc.), thiazolyl (e.g., 2-, 4- or 5-thiazolyl, etc.), pyrazolyl (e.g., 3-, 4- or 5-pyrazolyl, etc.), pyrrolidinyl (e.g., 2- or 3-pyrrolidinyl, etc.), imidazolyl (e.g., 2-, 4- or 5-imidazolyl, etc.), imidazolinyl (e.g., 2-imidazolinyl, 4-imidazolidinyl, etc.), isoxazolyl (e.g., 3-, 4- or 5-isoxazolyl, etc.), isothiazolyl (e.g., 3-, 4- or 5-isothiazolyl, etc.), oxadiazolyl [e.g., 3-or 5-(1,2,4-oxadiazolyl), 2-, 5- or 6-(1,3,4-oxadiazolyl), etc.], thiadiazolyl [e.g., 3- or 5-(1,2,4-thiadiazolyl) , 2- or 5-(1,3,4-thiadiazolyl), 4- or 5-(1,2,3-thiadiazolyl) , 3- or 4-(1,2,5-thiadiazolyl), etc.], and triazolyl [e.g., 2- or 5-(1,2,3-triazolyl), 3- or 5-(1,2,4-triazolyl), etc.], tetrazolyl [e.g., 5-(1H- or 2H-tetrazolyl), etc.]; (2) a 6-membered heterocyclic group containing 1 to 4 hetero atoms selected from oxygen atoms, sulfur atoms, nitrogen atoms etc., in addition to carbon atoms, and having a bond in a carbon atom thereof, such as pyridyl (e.g., 2-, 3- or 4-pyridyl, etc.), pyrimidinyl (e.g., 2-, 4- or 5-pyrimidinyl, etc.), thiomorpholinyl (e.g., 2- or 3-thiomorpholinyl, etc.), morpholinyl (e.g., 2- or 3-morpholinyl, etc.), triazinyl (e.g., 3- or 6-triazinyl, etc.), piperidinyl (e.g., 2-, 3- or 4-piperidinyl, etc.), pyranyl (e.g., 2- or 3-pyranyl, etc.), thiopyranyl (e.g., 2- or 3-thiopyranyl, etc.), oxazinyl [e.g., 2- or 3-(1,4-oxazinyl), etc.], thiazinyl [e.g., 2- or 3-(1,4-thiazinyl), 1- or 4-(1,3-thiazinyl), etc.], piperazinyl (e.g., 2- or 3-piperazinyl, etc.), triazinyl (e.g., 3- or 6-triazinyl, etc.), pyridazinyl (e.g., 3- or 4-pyridazinyl, etc.), pyrazinyl (e.g., 2- or 3-pyrazinyl, etc.), pyridazinyl (e.g., 3- or 4-pyridazinyl, etc.); and (3) a bicyclic or tricyclic condensed heterocyclic group containing 1 to 4 hetero atoms selected from oxygen atoms, sulfur atoms, nitrogen atoms etc., in addition to carbon atoms, and having a bond in a carbon atom thereof, such as benzofuryl, benzothiazolyl, benzoxazolyl, tetrazolo[1,5-b]pyridazinyl, triazolo[4,5-b]pyridazinyl, benzimidazolyl, quinolyl, isoquinolyl, cinnolinyl, phthalazinyl, quinazolinyl, quinoxalinyl, indolizinyl, indolyl, quinolizinyl, 1,8-naphthylidinyl, purinyl, pteridinyl, dibenzofuranyl, carbazolyl, acridinyl, phenanthridinyl, chromanyl, benzoxazinyl, phenazinyl, phenothiazinyl and phenoxazinyl, and the like.

The substituents in the heterocyclic group having a bond in a carbon atom thereof, which may be substituted is exemplified by the same substituents recited as examples of the above-described heterocyclic group which may be substituted.

The cyclic amino group and the cyclic amino group in the cyclic amino group which may be substituted is exemplified by 5- to 7-membered nitrogen-containing cyclic groups which may have an additional atom selected from oxygen atoms, sulfur atoms and nitrogen atoms. Examples of such groups include pyrrolidinyl, pyrrolinyl, pyrrolyl, pyrazolidinyl, pyrazolinyl, pyrazolyl, imidazolidinyl, imidazolinyl, imidazolyl, 1,2,3-triazinyl, 1,2,3-triazolidinyl, 1,2,3-triazolyl, 1,2,3,4-tetrazolyl, piperidinyl, piperazinyl, azepinyl, hexamethyleneimino, oxazolidino, morpholino, thiazolidino and thiomorpholino and the like. Of these, preferred is 5- to 6-membered cyclic amino group, such as pyrrolidinyl, pyrazolinyl, pyrazolyl, piperidinyl, piperazinyl, morpholino and thiomorpholino.

The cyclic amino group may have 1 to 3 substituents at any substitutable positions, such substituents including, for example, (1) C₁₋₆ alkyl (e.g. , methyl, ethyl, propyl, isopropyl, butyl, isobutyl, sec-butyl, tert-butyl (i.e., C₁₋₄ alkyl), pentane, hexane etc.), (2) C₆₋₁₄ aryl (e.g. , phenyl, naphthyl, anthryl, phenanthryl, acenaphthyl etc.), (3) C₇₋₁₀ aralkyl (phenyl-C₁₋₄ alkyl (e.g., benzyl, phenethyl etc.)), (4) benzhydryl, (5) C₁₋₆ alkyl-carbonyl (e.g., acetyl, propionyl etc.) , (6) C₆₋₁₄ aryl-carbonyl (e.g. , benzoyl etc.) , (7) C₁₋₆ alkoxy-carbonyl (e.g., methoxycarbonyl, ethoxycarbonyl, propoxycarbonyl, isopropoxycarbonyl, butoxycarbonyl, isobutoxycarbonyl, sec-butoxycarbonyl, tert-butoxycarbonyl, pentyloxycarbonyl, hexyloxycarbonyl etc.), and the like. Preferred substituent is C₁₋₆ alkyl, more preferred substituent is C₁₋₃ alkyl.

The homocyclic group of the homocyclic group which may be substituted is exemplified by 3- to 7-membered carbocyclic groups which may be condensed, such as C₆₋₁₀ aryl groups (e.g., phenyl, naphthyl, etc.), C₃₋₇ cycloalkyl groups (e.g., cyclopropyl, cyclobutyl, cyclopentyl, cyclohexyl, cycloheptyl, etc.) and C₃₋₇ cycloalkenyl (e.g., cyclopropenyl, cyclobutenyl, cyclopentenyl, cyclohexenyl, cycloheptenyl, etc.), and the like.

Such homocyclic groups may have 1 to 6, preferably 1 to 3, and more preferably 1 or 2, substituents at any substitutable positions. Such substituents include, for example, (1) C₁₋₁₅ alkyl which may be substituted by 1 to 3, preferably 1 or 2, halogens (e.g., fluorine, chlorine, bromine, iodine etc.) (e.g., methyl, ethyl, propyl, isopropyl, butyl, isobutyl, sec-butyl, tert-butyl, pentyl, hexyl (i.e., C₁₋₆ alkyl), heptyl, octyl, nonyl, decyl, undecyl, dodecyl, tridecyl, tetradecyl, pentadecyl etc.), preferably C₁₋₆ alkyl which may be substituted by halogen, (2) C₃₋₁₀ cycloalkyl (e.g., cyclopropyl, cyclobutyl, cyclopentyl, cyclohexyl, cycloheptyl, cyclooctyl, cyclononyl, cyclodecyl etc.) , (3) C₂₋₁₀ alkenyl (e.g., vinyl, allyl, isopropenyl, 1-butenyl, 2-butenyl, 3-butenyl, butadienyl, 2-methylallyl, hexatrienyl, 3-octenyl etc.), (4) C₂₋₁₀ alkynyl (e.g., ethynyl, 2-propynyl, butynyl, 3-hexynyl etc.), (5) C₃₋₁₀ cycloalkenyl (e. g., cyclopropenyl, cyclopentenyl, cyclohexenyl etc.), (6) C₆₋₁₀ aryl (e.g., phenyl, naphthyl etc.), (7) C₇₋₂₀ aralkyl (e.g., benzyl, phenethyl etc.), (8) nitro, (9) hydroxy, (10) mercapto, (11) oxo, (12) thioxo, (13) cyano, (14) carbamoyl, (15) carboxyl, (16) C₁₋₆ alkoxy-carbonyl (e.g., methoxycarbonyl, ethoxycarbonyl, etc.), (17) sulfo, (18) halogen (e.g., fluorine, chlorine, bromine, iodine etc.), (19) C₁₋₆ alkoxy (e.g., methoxy, ethoxy, propoxy, isopropoxy, butoxy, isobutoxy, sec-butoxy, tert-butoxy, pentyloxy, hexyloxy etc.), (20) C₆₋₁₀ aryloxy (e.g., phenoxy, etc.), (21) C₁₋₆ acyloxy (e.g., C₁₋₆ alkanoyloxy such as acetoxy and propionyloxy, etc.), (22) C₁₋₆ alkylthio (e.g., methylthio, ethylthio, propylthio, isopropylthio, butylthio, tert-butylthio, etc.), (23) C₆₋₁₀ arylthio (e.g., phenylthio, etc.), (24) C₁₋₆ alkylsulfinyl (e.g., methylsulfinyl, ethylsulfinyl, etc.), (25) C₆₋₁₀ arylsulfinyl (e.g., phenylsulfinyl, etc.), (26) C₁₋₆ alkylsulfonyl (e.g., methylsulfonyl, ethylsulfonyl, etc.), (27) C₆₋₁₀ arylsulfonyl (e.g., phenylsulfonyl, etc.), (28) amino, (29) C₁₋₆ acylamino (e.g., C₁₋₆ alkanoylamino such as acetylamino, propionylamino, etc.), (30) mono- or di-C₁₋₄ alkylamino (e.g., methylamino, ethylamino, propylamino, isopropylamino, butylamino, dimethylamino, diethylamino, etc.), (31) C₃₋₈ cycloalkylamino (e.g., cyclopropylamino, cyclobutylamino, cyclopentylamino, cyclohexylamino, etc.), (32) C₆₋₁₀ arylamino (e.g., anilino, etc.), (33) C₁₋₆ alkanoyl (e.g., formyl, acetyl, hexanoyl, etc.), (34) C₆₋₁₀ aryl-carbonyl (e.g., benzoyl, etc.), and (35) 5- or 6-membered heterocyclic groups containing 1 to 4 hetero atoms selected from oxygen, sulfur, nitrogen, etc., in addition to carbon atoms [e.g., thienyl (e.g., 2- or 3-thienyl, etc.), furyl (e.g., 2- or 3-furyl, etc.), pyrazolyl (e.g., 3-, 4- or 5-pyrazolyl, etc.), thiazolyl (e.g., 2-, 4- or 5-thiazolyl, etc.), isothiazolyl (e.g., 3-, 4- or 5-isothiazolyl, etc.), oxazolyl (e.g., 2-, 4-or 5-oxazolyl, etc.), isoxazolyl (e.g., 3-, 4- or 5-isoxazolyl, etc.), imidazolyl (e.g., 2-, 4- or 5-imidazolyl, etc.), triazolyl (e.g., 1,2,3- or 1,2,4-triazolyl, etc.), tetrazolyl (e.g., 1H- or 2H-tetrazolyl, etc.), pyridyl (e.g., 2-, 3- or 4-pyridyl, etc.), pyrimidinyl (e.g., 2-, 4- or 5-pyrimidyl, etc.), pyridazinyl (e.g., 3- or 4-pyridazinyl, etc.), quinolyl, isoquinolyl, indolyl, etc.], and the like.

The hydroxy group which may be substituted for R²¹ and R²³ includes, for example, a group of the above-mentioned formula: -OR²⁶ wherein R²⁶ is as defined above.

In the formula, R¹⁴, R¹⁵ and R¹⁶ are the same or different and each is preferably (i) a hydrogen or (ii) the above-described group bound via a carbon atom, a nitrogen atom or an oxygen atom. Preference is given to the case wherein R¹⁴ is a C₁₋₁₅ alkyl group which may be substituted, a C₃₋₁₀ cycloalkyl group which may be substituted, a C₂₋₁₀ alkenyl group which may be substituted, a C₂₋₁₀ alkynyl group which may be substituted, a C₃₋₁₀ cycloalkenyl group which may be substituted, a C₆₋₁₄ aryl group which may be substituted, a C₇₋₂₀ aralkyl group which may be substituted, a C₁₋₂₀ acyl group which may be substituted, a nitro group, a group of the formula: -NR²³R²⁴ wherein R²³ and R²⁴ are as defined above), or a group of the formula: -O-R²⁶ wherein R²⁶ is a hydrogen atom or a C₁₋₁₀ hydrocarbon group which may be substituted, a C₁₋₂₀ acyl group which may be substituted, a C₁₋₂₀ alkylsulfonyl group which may be substituted, a C₆₋₁₄ arylsulfonyl group which may be substituted or a 5- to 8-membered heterocyclic group which may be substituted (same as the above-described "5- to 8-membered heterocyclic group containing 1 to 4 hetero atoms selected from oxygen atoms, sulfur atoms, nitrogen atoms etc., in addition to carbon atoms"), wherein at least one of R¹⁵ and R¹⁶ is a hydrogen, and the other is the above-described group bound via a carbon atom, a nitrogen atom, or an oxygen atom, preferably R¹⁵ and R¹⁶ are both hydrogens.

R¹⁴ is preferably a C₁₋₁₀ alkyl group (preferably a C₁₋₆ alkyl group) which may be substituted by 1 to 3, preferably 1, hydroxy group, a nitro group, an amino group, the formula: - NR²³R²⁴ [wherein R²³ represents a hydrogen; R²⁴ represents C₁₋₆ alkyl-carbonyl which may be substituted by 1 to 3, preferably 1, hydroxy group, C₁₋₆ alkylamino-carbonyl which may be substituted by 1 to 3, preferably 1, C₁₋₆ alkoxy group (e.g., methoxy, ethoxy, propoxy, isopropoxy, butoxy, isobutoxy, sec-butoxy, tert-butoxy, pentyloxy, hexyloxy etc.), or C₆-₁₄ arylamino-carbonyl], or the formula: -O-R²⁵ wherein R²⁶ represents a hydrogen, C₁₋₁₀ alkyl which may be substituted by 1 to 3, preferably 1, hydroxy, a C₁₋₆ alkyl-carbonyl which may be substituted by C₃₋₁₀ cycloalkyl or 1 to 3, preferably 1, hydroxy, a C₁₋₆ alkylsulfonyl group, or a C₆₋₁₀ arylsulfonyl group.

In the formula, R¹⁷ is preferably (1) a C₁₋₁₀ hydrocarbon group which may be substituted, (2) a C₁₋₂₀ acyl group which may be substituted, (3) a heterocyclic group having a bond in a carbon atom thereof which may be substituted, (4) a carboxyl group which may be esterified or amidated, or (5) a cyano group. More preferably, R¹⁷ is a C₁₋₁₅ alkyl group which may be substituted, a C₃₋₁₀ cycloalkyl group which may be substituted, a C₂₋₁₀ alkenyl group which may be substituted, a C₂₋₁₀ alkynyl group which may be substituted, a C₃₋₁₀ cycloalkenyl group which may be substituted, a C₆₋₁₄ aryl group which may be substituted or a C₇₋₂₀ aralkyl group which may be substituted. Still more preferred is a C₁₋₆ alkyl group which may be substituted such as an aminoalkyl group which may be substituted, and the like. A preferred example of R¹⁷ is the formula: -(CH₂) ᵣ-NR²³R²⁴ wherein r is an integer of 1 to 3; R²³ is hydrogen, a C₁₋₁₀ hydrocarbon group which may be substituted, a C₁₋₂₀ acyl group which may be substituted, a hydroxy group which may be substituted (group of the above-described formula: -O-R²⁶), a heterocyclic group which may be substituted, or a group of the formula: -S(O)ₜ-R²⁵ wherein t is an integer of 0 to 2; R²⁵ is a hydrogen atom or a C₁₋₁₀ hydrocarbon group which may be substituted; R²⁴ is hydrogen or a C₁₋₁₀ hydrocarbon group; or R²³ and R²⁴ may form, taken together with the adjacent nitrogen atom, a cyclic amino group which may be substituted. R¹⁷ is more preferably a C₁₋₃ alkyl group which may be substituted by a halogen atom, a hydroxy group which may be substituted by a C₁₋₂₀ acyl group, or an amino group which may be substituted by C₁₋₁₀ alkyl and/or C₆₋₁₄ aryl-C₁₋₁₀ alkyl. R¹⁷ is particularly preferably N-C₁₋₆ alkyl-N-benzylaminomethyl.

In the formula, the halogen represented by R¹⁸ is exemplified by fluorine, chlorine, bromine and iodine.

R¹⁸ is preferably a hydrogen, a C₁₋₁₅ alkyl group which may be substituted, a C₃₋₁₀ cycloalkyl group which may be substituted, a C₂₋₁₀ alkenyl group which may be substituted, a C₂₋₁₀ alkynyl group which may be substituted, a C₃₋₁₀ cycloalkenyl group which may be substituted, a C₆₋₁₄ aryl group which may be substituted, a C₇₋₂₀ aralkyl group which may be substituted, a C₁₋₂₀ acyl group which may be substituted, a carboxyl group which may be esterified or amidated, or the formula: -O-R²⁶ wherein R²⁶ is a hydrogen atom, a C₁₋₁₅ alkyl group which may be substituted, a C₃₋₁₀ cycloalkyl group which may be substituted, a C₂₋₁₀ alkenyl group which may be substituted, a C₂₋₁₀ alkynyl group which may be substituted, a C₃₋₁₀ cycloalkenyl group which may be substituted, a C₆₋₁₄ aryl group which may be substituted, a C₇₋₂₀ aralkyl group which may be substituted, a C₁₋₂₀ acyl group which may be substituted, a C₁₋₂₀ alkylsulfonyl group which may be substituted, a C₆₋₁₄ arylsulfonyl group which may be substituted or a heterocyclic group which may be substituted. Of these, preferred examples of R¹⁸ include a hydrogen, a C₁₋₁₅ alkyl group which may be substituted by 1 to 3, preferably 1, C₆₋₁₄ aryl or C₁₋₆ alkoxy group, or a C₁₋₆ alkyl-carbonyl which may be substituted by 1 to 3, preferably 1, hydroxy group, C₁₋₆ alkoxycarbonyl (e.g., methoxycarbonyl, ethoxycarbonyl, isopropoxycarbonyl, tert-butoxycarbonyl, etc.), C₆₋₁₄ aryl-carbonyl (e.g., benzoyl, etc.), C₆₋₁₄ aryloxy-carbonyl (e.g. , phenoxycarbonyl, etc.), C₇₋₁₅ aralkyl-carbonyl (e.g., benzylcarbonyl, etc.), C₇₋₁₉ aralkyloxy-carbonyl (e.g. , benzyloxycarbonyl, etc.) , N-C₁₋₁₀ alkyl-N- (C₁₋₁₀ alkoxy)amino-carbonyl (e.g., N-methyl-N-methoxyamino-carbonyl, etc. ) , C₁₋₁₅ alkyloxy or C₁₋₂₀ arylsulfonyl group, and the like. R¹⁸ is more preferably (1) a C₁₋₆ alkoxy-carbonyl group, (2) a C₆₋₁₄ aryl group which may be substituted by halogen or C₁₋₆ alkoxy, or (3) a phenyl-C₁₋₃ alkyl group.

In the formula, R¹⁹ is preferably a hydrogen, a C₁₋₁₅ alkyl group which may be substituted, a C₃₋₁₀ cycloalkyl group which may be substituted, a C₂₋₁₀ alkenyl group which may be substituted, a C₂₋₁₀ alkynyl group which may be substituted, a C₃₋₁₀ cycloalkenyl group which may be substituted, a C₆₋₁₄ aryl group which may be substituted or a C₇₋₂₀ aralkyl group which may be substituted. More preferably, R¹⁹ is a hydrogen or a C₁₋₁₀ alkyl group. Still more preferably, R¹⁹ is a hydrogen or a C₁₋₆ alkyl group.

In the formula, R²⁰ is a homocyclic group which may be substituted or a heterocyclic group which may be substituted, preferably a C₆₋₁₄ aryl group which may be substituted. More preferably, R²⁰ is a phenyl group which may be substituted by 1 to 3, preferably 1 or 2, halogen atoms or C₁₋₆ alkoxy group. Particularly preferred is a phenyl group which may be substituted by 1 or 2 halogen atoms.

In the above formula (XII), m is an integer of 0 to 3, preferably 0 to 2, and more preferably 0 or 1.

In the above formula, r is an integer of 1 to 3, preferably 1 or 2, and more preferably 1.

In the above formula (XII), one of A and D represents a nitrogen atom and the other represents a carbon atom, or both represent nitrogen atom (s); B represents a nitrogen atom or a carbon atom. Compounds represented by the formula (XII) are therefore exemplified by compounds represented by the following formulas: 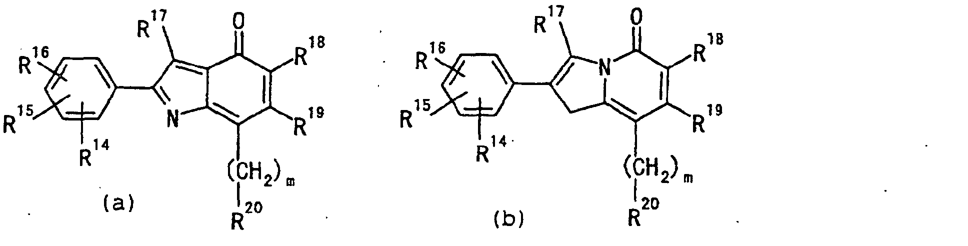 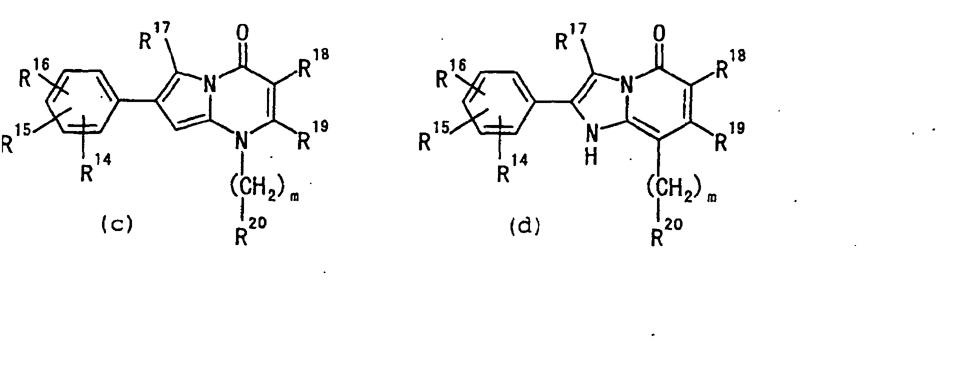 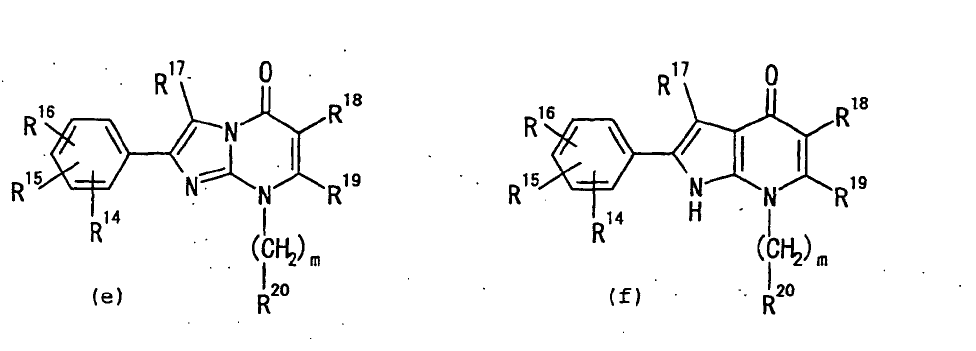 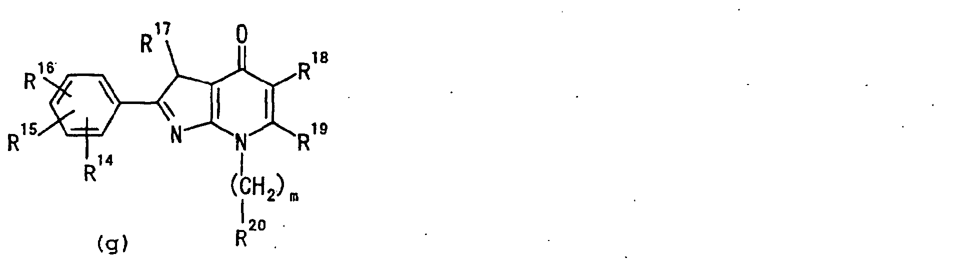 wherein each symbol is as defined above, preferably compounds represented by the formulas (a), (b), (c), (d), (e) and (g). Of these, preferred is a compound of the formula (XII) wherein B is a nitrogen atom, particularly preferred is a compound represented by the formulas (c) and (e), and most preferred is a compound represented by the formula (e).

In compound (XII), preferred is a compound of the formula: 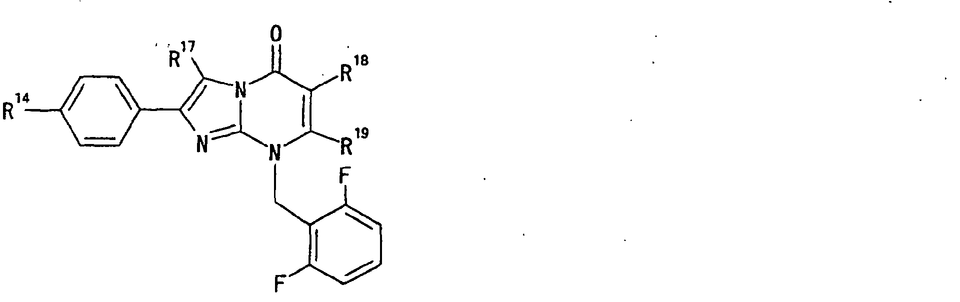 wherein each symbol is as defined above. Of the compound (XII), more preferred is a compound wherein R¹⁴ is (1) an amino group which may be substituted by (i) carbamoyl which may be substituted by C₁₋₆ alkyl or C₁₋₆ alkoxy, or (ii) C₁₋₆ alkyl-carbonyl, or (2) a C₁₋₆ alkoxy group which may be substituted by C₃₋₆ cycloalkyl;
R¹⁷ is an N-C₁₋₆ alkyl-N-benzylaminomethyl group;
R¹⁸ is (1) a C₁₋₆ alkoxy-carbonyl group, (2) a C₆₋₁₄ aryl group which may be substituted by halogen or C₁₋₆ alkoxy, or (3) a phenyl-C₁₋₃ alkyl group; and
R¹⁹ is a hydrogen atom.

In addition, a compound wherein R¹⁴ is (1) a nitro group, (2) an amino group which may be substituted by 1 or 2 substituents selected from (i) C₁₋₆ alkyl which may be substituted by hydroxy, (ii) C₁₋₆ alkyl-carbonyl which may be substituted by hydroxy, halogen or thienyl, (iii) C₆₋₁₀ aryl-carbonyl which may be substituted by C₁₋₆ alkyl, C₁₋₆ alkoxy or halogen, (iv) C₃₋₆ cycloalkyl-carbonyl, (v) C₂₋₄ alkenyl-carbonyl, (vi) C₁₋₆ alkoxy-carbonyl, (vii) C₁₋₆ alkylamino- carbonyl, (viii) C₁₋₆ alkoxyamino-carbonyl, (ix) phenylaminocarbonyl, (x) isoxazolylcarbonyl, thienylcarbonyl, thiazolylcarbonyl, pyrazolylcarbonyl or furylcarbonyl, which may be substituted by 1 or 2 substituents selected from C₁₋₆ alkyl, nitro and C₁₋₆ alkoxy, (xi) pyridylcarbonyl, (xii) C₁₋₆ alkylsulfonyl, (xiii) thienylsulfonyl and (xiv) phenylsulfonyl which may be substituted by C₁₋₆ alkyl,
(3) a pyrrolyl group or
(4) a hydroxy group which may be substituted by C₁₋₆ alkyl, C₃₋₆ cycloalkyl-C₁₋₃ alkyl or C₁₋₆ alkyl-carbonyl;
R¹⁷ is a C₁₋₆ alkyl group which may be substituted by 1 or 2 substituents selected from (1) halogen, (2) hydroxy and (3) amino which may be substituted by 1 or 2 substituents selected from C₁₋₆ alkyl, phenyl-C₁₋₃ alkyl and di-C₁₋₆ alkylamino-C₁₋₃ alkyl;
R¹⁸ is (1) halogen, (2) a phenyl group which may be substituted by halogen or C₁₋₆ alkyl, or (3) a carbonyl group substituted by (i) C₁₋₆ alkyl, (ii) amino substituted by C₁₋₆ alkyl and C₁₋₆ alkoxy or (iii) C₁₋₆ alkoxy; and
R¹⁹ is a hydrogen atom or a C₁₋₃ alkyl group, is also preferable.

As compound (XII), concretely mentioned are 8-(2,6-difluorobenzyl)-5,8-dihydro-2-[4-(ethylaminocarbonylamino)phenyl]-3-(N-methyl-N-benzylaminomethyl)-5-oxoimidazo[1,2-a]pyrimidine-6-carboxylic acid ethyl ester, 8-(2,6-difluorobenzyl)-5,8-dihydro-2-[4-(methoxyaminocarbonylamino)phenyl]-3-(N-methyl-N-benzylaminomethyl)-5-oxoimidazo[1,2-a]pyrimidine-6-carboxylic acid isopropyl ester, 8-(2,6-difluorobenzyl)-5,8-dihydro-2-[4-(ethylaminocarbonylamino)phenyl]-3-(N-methyl-N-benzylaminomethyl)-5-oxoimidazo[1,2-a]pyrimidine-6-carboxylic acid isopropyl ester, salts thereof, and the like.

Compound (XII) and a salt thereof can be produced by, for example, a known method such as a method disclosed in JP-A-11-315079, WO99/33831 or a method analogous thereto.

Of the embodiments of compounds shown in the above-mentioned [I] - [IV], compound (I) shown in [I] and a salt thereof are preferable.

As the "physiologically active non-peptide substance", moreover, somatostatin receptor (SSTR) agonist or antagonist, preferably SSTR agonist, can be mentioned. While SSTR includes subtypes of types 1, 2, 3, 4 and 5, type 2 SSTR (SSTR 2) agonist is preferable.

The "SSTR 2 agonist" may be any compound as long as it shows SSTR 2 agonism, and, for example, the embodiment of compound (XIII) and the like can be mentioned.

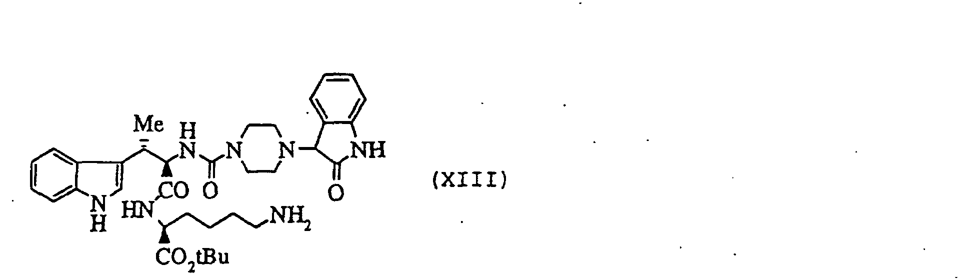

Compound (XIII) is tert-butyl (2S)-6-amino-2-{[(2R,3S)-3-(1H-indol-3-yl)-2-({[4-(2-oxo-2,3-dihydro-1*H*-benzimidazol-3-yl)-1-piperidinyl]carbonyl)amino)butanoyl]amino}hexanoate.

Compound (XIII) is synthesized by the method described in Proceedings of the National Academy of Sciences (1998), vol. 95, pp. 10836-10841.

As the "physiologically active non-peptide substance", a substance having a molecular weight of about 1,000 or below, preferably about 10 or above and about 900 or below, more preferably'about 50 or above and about 900 or below, more preferably about 50 or above and about 800 or below, still more preferably about 100 or above and about 800 or below, particularly preferably about 100 or above and about 700 or below is preferable.

As the "organic acid" to be used in the present invention, for example, adipic acid, fumaric acid, as well as lower fatty acid, aliphatic sulfonic acid, aliphatic hydroxycarboxylic acid, aromatic organic acid (e.g., aromatic carboxylic acid, aromatic hydroxycarboxylic acid, aromatic sulfonic acid etc.) and the like can be mentioned. As the lower fatty acid, for example, acetic acid, propionic acid, butyric acid and the like can be mentioned. As the aliphatic sulfonic acid, for example, methanesulfonic acid and the like can be mentioned. As the aliphatic hydroxycarboxylic acid, for example, lactic acid, tartaric acid, malic acid, citric acid and the like can be mentioned. As the aromatic carboxylic acid, for example, benzoic acid, phthalic acid and the like can be mentioned. As the aromatic hydroxycarboxylic acid, for example, salicylic acid, hydroxynaphthoic acid (e.g., 1-hydroxy-2-naphthoic acid, 3-hydroxy-2-naphthoic acid), pamoic acid and the like can be mentioned. As the aromatic sulfonic acid, for example, benzenesulfonic acid and the like can be mentioned.

Of the above-mentioned organic acids, lower fatty acid, aliphatic hydroxycarboxylic acid and aromatic organic acid are preferable.

The "organic acid" is produced by a known method or a method analogous thereto.

The hydroxynaphthoic acid to be used in the present invention has naphthalene and one hydroxy group and one carboxyl group each attached to different carbons in the naphthalene. Accordingly, a total of 14 kinds of isomers having different position of hydroxy group is present with respect to 1-carboxyl group and 2-carboxyl group of each naphthalene ring. An optional isomer therefrom may be used, and a mixture of these isomers at an optional ratio may be also used. As mentioned below, one having a greater acid dissociation constant, i.e., smaller pKa (pKa=-log₁₀ Ka, Ka is acid dissociation constant), is preferable. Furthermore, one that is slightly water-soluble is preferable.

As the pKa value of the isomers of the above-mentioned hydroxynaphthoic acids, only the value of 3-hydroxy-2-naphthoic acid (pKa=2.708, Handbook of Chemistry, Basic II, The Chemical Society of Japan, September 25, 1969 issue) is known. However, a useful finding is obtained by comparison of the pKa values of three kinds of isomers of hydroxybenzoic acid. That is, the pKa of m-hydroxybenzoic acid and p-hydroxybenzoic acid is 4 or above, but the pKa (=2.754) of o-hydroxybenzoic acid (salicylic acid) is extremely small. Accordingly, among the above-mentioned 14 kinds of isomers, 3-hydroxy-2-naphthoic acid, 1-hydroxy-2-naphthoic acid and 2-hydroxy-1-naphthoic acid, wherein a carboxyl group and a hydroxy group are attached to the vicinal carbon atoms in the naphthalene ring, are preferable.

The "aromatic hydroxycarboxylic acid" to be used in the present invention may be a mixture of various aromatic hydroxycarboxylic acids. For example, combinations of salicylic acid and 3-hydroxy-2-naphthoic acid, salicylic acid and pamoic acid, 3-hydroxy-2-naphthoic acid and pamoic acid, 1-hydroxy-2-naphthoic acid and pamoic acid, 2-hydroxy-1-naphthoic acid and pamoic acid, or salicylic acid and 3-hydroxy-2-naphthoic acid and pamoic acid and the like, and the like can be mentioned, with preference given to a combination of 3-hydroxy-2-naphthoic acid and pamoic acid and a combination of 1-hydroxy-2-naphthoic acid and pamoic acid.

The "biocompatible organic solvent" to be used in the present invention may be any as long as it can be generally added to pharmaceutical products (organic solvents described in Pharmaceutical Excipients Dictionary 2000, edited by Japan Pharmaceutical Excipients Council, YAKUJI NIPPO LTD. and those analogous thereto). As the organic solvent, those that easily dissolve the non-peptide substances are preferable. The biocompatible organic solvents to be used in the present invention are recited in the following, but such solvents are not limited to the following.

As examples of the biocompatible organic solvents, α-thioglycerin, dimethyl sulfoxide, glycerol, lower alcohol (e.g., methanol, ethanol, ethanol anhydride, isopropanol etc.), lauric acid, ethylene glycol, polyethylene glycol (e.g., polyethylene glycol 200, polyethylene glycol 300, polyethylene glycol 400, polyethylene glycol 600), ethylenediamine, m-cresol, oleic acid diethanolamine, N,N-dimethylacetamide, thioglycolic acid, caproic acid, triethanolamine, propylene glycol, monoethanolamine, caprylic acid, 2-ethylhexyl acrylate - vinylpyrrolidone copolymer solution, capric acid, 2-ethylhexyl acrylate - 2-ethylhexyl methacrylate - dodecyl methacrylate copolymer solution, linoleic acid, isostearyl alcohol, isostearic acid, allyl isothiocyanate, 2-ethyl-1,3-hexanediol, oleyl alcohol, chletamitone, geraniol, dipropylene glycol, ethers (e.g., diethylene glycol monoethyl ether, dimethyl ether, polyoxyethylene octylphenyl ether, α-monoisostearyl glyceryl ether etc.), dimethylpolysiloxane, cinnamaldehyde, petroleum benzine, tocopherol, triethylene glycol, trichloroethane, benzyl nicotinate, butylphthalyl butylglycolate, 1,3-butyleneglycol, hexydecanol, ketones (e.g., methylisobutylketone, methylethylketone etc.), N-methyl-2-pyrrolidone, lauryl alcohol, lauromacrogol, lanolin alcohol and the like can be mentioned.

In addition, fatty acid esters such as ethyl acetate, benzyl acetate, hexyl laureate, isopropyl myristate, octyldodecyl myristate, decyl oleate, isostearyl palmitate, hexadecyl isostearate, ethyl linoleate, diisobutyl adipate, diisopropyl adipate, isoamyl isovalerate, diisopropyl sebacate, cetyl 2-ethylhexanoate and the like, aromatic fatty acid esters such as benzyl benzoate, diethyl phthalate, dioctyl phthalate and the like, and the like can be mentioned.

In addition, glycerin fatty acid esters such as vegetable oil, triacetin, triglyceride of medium chain fatty acid, glycerin dioleate, glycerin monoisostearate, glycerin monooleate and the like, propylene glycol fatty acid esters such as propylene glycol monocaprylate, propylene glycol monostearate, propylene glycol dicaprate, propylene glycol dicaprylate, propylene glycol caprylate caprate and the like, and the like can be mentioned.

In addition, polyglycerine fatty acid esters such as diglyceryl monooleate, diglyceryl dioleate, diglyceryl monoisostearate, diglyceryl monooleate, tetraglyceryl monooleate, tetraglyceryl pentaoleate, hexaglyceryl monolaurate, hexaglyceryl monomyristate, hexaglyceryl monooleate, hexaglyceryl pentaoleate, hexaglyceryl polyricinoleate, decaglyceryl monolaurate, decaglyceryl monooleate, decaglyceryl monolinolate, decaglyceryl trioleate, decaglyceryl pentaoleate, decaglyceryl heptaoleate, decaglyceryl decaoleate and the like, polyethylene glycol fatty acid esters such as glycerin polyethylene glycol caproate caprylate, polyoxyethylene glycerol fatty acid ester, polyethylene glycol monooleate, polyethylene glycol dioleate, polyethylene glycol dilinolate and the like, polyoxyethylene castor oil, polyoxyethylene hydrogenated castor oil and the like can be mentioned.

In addition, sorbitan fatty acid esters such as sorbitan monolaurate, sorbitan monooleate, sorbitan sesquioleate, sorbitan trioleate and the like, polyoxyethylene sorbitan fatty acid esters such as polysorbate 20, polysorbate 40, polysorbate 60, polyoxyethylene sorbitan monooleate and the like, polyoxyethylene sorbitan fatty acid esters such as polyoxyethylene sorbitan monolaurate, polyoxyethylene sorbitan tetraoleate and the like, and the like can be mentioned.

Of those mentioned above, polyethylene glycol, fatty acid ester thereof, and dimethyl sulfoxide are preferable.

For the biocompatible organic solvent, two or more kinds (preferably 1 to 5 kinds, more preferably 1 to 3 kinds) of those mentioned above may be used in a mixture.

As the hydrophilic polymer to be used in the present invention, for example, hydroxyalkyl cellulose such as hydroxypropyl cellulose, hydroxypropylmethyl cellulose and the like, alkyl cellulose such as methyl cellulose, ethyl cellulose and the like, polyalkenylpyrrolidone such as polyvinylpyrrolidone and the like, aminoalkylmethacrylate copolymer E, polyvinylacetal diethylaminoacetate and the like can be mentioned.

In addition, hydrophilic polymers such as carboxyvinyl polymer, polyvinyl alcohol, gum arabic, sodium alginate, propylene glycol alginate, agar, gelatin and chitosan can be mentioned.

For the hydrophilic polymer, two or more kinds (preferably 1 to 5 kinds, more preferably 1 to 3 kinds) of those mentioned above may be used in a mixture.

The content of the "physiologically active non-peptide substance" in the "pharmaceutical solution" of the present invention is preferably about 5 wt% or above; for example, about 5 wt% to about 90 wt%, preferably about 10 wt% to about 80 wt%, more preferably about 20 wt% to about 70 wt%, relative to the total weight of the solution.

The content of the "organic acid" in the "pharmaceutical solution" of the present invention is, for example, about 0.1 wt% to about 50 wt%, preferably about 1 wt% to about 40 wt%, more preferably about 5 wt% to about 30 wt%, relative to the total weight of the solution.

The content of the "biocompatible organic solvent" in the "pharmaceutical solution" of the present invention is, for example, about 30 wt% to about 94.9 wt%, preferably about 40 wt% to about 89 wt%, more preferably about 50 wt% to about 75 wt%, relative to the total weight of the solution.

The amount ratio of the physiologically active non-peptide substance and the organic acid is, for example, about 1/20 to about 100 moles, preferably about 1/10 to about 20 moles, more preferably about 1/5 to about 10 moles of the organic acid, per 1 mole of the physiologically active non-peptide substance.

The solubility of the "physiologically active non-peptide substance" in a biocompatible organic solvent means a drug concentration in a filtrate obtained by adding an excess amount of a drug to a biocompatible organic solvent, agitating the mixture using, for example, a recipro shaker (model SR-I, Taiyo Scientific Industrial Co., Ltd.) at 100 times or more per minute at room temperature (about 15°C to about 25°C) for 30 minutes or longer and filtering the mixture.

In the present invention, "the physiologically active non-peptide substance is contained at a concentration higher than the solubility of the physiologically active non-peptide substance in the biocompatible organic solvent" means that the concentration of the physiologically active non-peptide substance in the present pharmaceutical solution is above the solubility, wherein, for example, the concentration is preferably not lower than about 1.2 times, more preferably not lower than about 1.5 times, still more preferably not less lower than about 2 times, the solubility.

The "pharmaceutical solution" of the present invention can be prepared according to a known method, by mixing a physiologically active non-peptide substance, an organic acid and a biocompatible organic solvent. For this mixing, the order of the physiologically active non-peptide substance, the organic acid and the biocompatible organic solvent may be optional. That is, after mixing the physiologically active non-peptide substance with the organic acid, the biocompatible organic solvent may be added to the mixture; after adding and mixing the physiologically active non-peptide substance to/with the biocompatible organic solvent, the organic acid may be added to the mixture and mixed; after adding and mixing the organic acid to/with the biocompatible organic solvent, the mixture may be added and mixed to/with the physiologically active non-peptide substance; and the like. For the mixing, any conditions of standing still, agitation, rotation, stirring and the like may be employed and the mixing may be done under heating, where necessary.

The "pharmaceutical solution" of the present invention may contain additives generally used for pharmaceutical products. For example, stabilizer, preservative, antioxidant, lubricant, surfactant, anticoagulant, thickener and the like can be mentioned. The pharmaceutical solution of the present invention may contain an organic acid or additive to be used where necessary in a dispersion state or suspension state, as long as the physiologically active non-peptide substance is dissolved. The "pharmaceutical solution" of the present invention preferably does not contain a biodegradable polymer.

The "pharmaceutical solution" of the present invention can be formulated into various dosage forms as it is or as a starting material, and administered as a preparation for parenteral administration, such as a preparation for injection (intramuscular injection, subcutaneous injection, injection to organs and the like) or a preparation for implantation, transmucosal drugs for nasal cavity, rectum, uterus and the like, a transdermal agent and the like, or as a preparation for oral administration such as capsules (e.g., hard capsule, soft capsule etc.), liquid such as syrup, emulsion, suspension and the like, and the like.

The "pharmaceutical solution" of the present invention may be a solution itself produced using only a physiologically active non-peptide substance, an organic acid and a biocompatible solvent, or can be prepared into a liquid, a candy, a gel or a paste with an additive to be used as necessary. In the case of a liquid, a sustained-release preparation, wherein the liquid has been charged in a sustained release pump, can be produced.

As the sustained release pump, OROS™ manufactured by ALZA Corporation for oral use can be mentioned. This is a system consists of a drug reservoir element and a polymer element that swells by osmotic pressure, wherein the polymer gradually swells to release the drug from the reservoir element. As the pump for injection, DUROS™ manufactured by ALZA Corporation for injection can be mentioned. Again, the drug is released from the injection site by the osmotic pressure, like OROS. Besides these, a pump using electric motor, spring motor, external energy utilization type motor (electromagnetic field, ultrasonic wave, microwave etc.), heat swellable polymer, photoreactive polymer, pH reactive polymer and the like as a driving force can be also used.

The size of the sustained release pump for oral administration is preferably 15 mL or less, more preferably 0.1 mL or above and 7 mL or less, in volume. The size of the sustained release pump for injection administration is preferably 8 mL or less, more preferably 0.1 mL or above and 6 mL or less, in volume. The volume of the reservoir in the sustained release pump for oral administration is preferably 7 mL or less, more preferably 0.01 mL or above and 3 mL or less. The volume of the reservoir in the sustained release pump for injection administration is preferably 4 mL or less, more preferably 0.01 mL or above and 2 mL or less.

The release period of the sustained release pump for oral administration is not less than 1 hour and not more than 48 hours, preferably not less than 2 hours and not more than 36 hours, more preferably not less than 3 hours and not more than 24 hours. When the sustained release pump is for injection, the release period is not less than 1 day and not more than 2 years, preferably not less than 1 week and not more than 1.5 years, more preferably not less than 2 weeks and not more than 1 year.

The pharmaceutical solution of the present invention is low toxic and can be used as a safe pharmaceutical agent to mammals (e.g., human, bovine, swine, dog, cat, mouse, rat, rabbit etc.).

The pharmaceutical solution of the present invention can be used as an agent for 'the prophylaxis or treatment of various diseases and the like, depending on the kind of the physiologically active substance contained therein. When the physiologically active substance is a GnRH antagonist, for example, the solution is useful for the prophylaxis and/or treatment of, for example, sex hormone-dependent cancers (e.g., prostate cancer, uterine cancer, breast cancer, pituitary tumor etc.), bone metastasis of sex hormone-dependent cancers, prostatomegaly, hysteromyoma, endometriosis, metrofibroma, precocious puberty, amenorrhea, premenstrual syndrome, dysmenorrhea, multilocular ovary syndrome, polycystic ovary syndrome, pimples, alopecia, Alzheimer's disease (Alzheimer's disease, Alzheimer's senile dementia and mixed type thereof) and the like. The pharmaceutical solution of the present invention is also useful for the regulation of reproduction in males and females (e.g., birth control agent, menstrual cycle regulator etc.). In addition, the pharmaceutical solution of the present invention can be used as a male or female contraceptive, and as a female ovulation inducer. The pharmaceutical solution of the present invention can be used for the treatment of infertility utilizing its rebound effect (recovery of hormone secretion) after cessation of the drug administration. In addition, it can be used as an agent for the prophylaxis or treatment of sex hormone non-dependent and LH-RH sensitive benign or malignant tumor and the like. Furthermore, the pharmaceutical solution of the present invention can be used as an agent for the prophylaxis or treatment of irritable bowel syndrome as well as sex hormone-dependent cancer postoperative recurrence preventive (prostatic cancer postoperative recurrence preventive, breast cancer or ovarian cancer postoperative recurrence preventive in premenopause and postmenopause).

In addition, the pharmaceutical solution of the present invention is useful for regulation of animal estrus, improvement of meat quality and promotion of animal growth in the field of animal husbandry, and the like. The pharmaceutical solution of the present invention is also useful as a fish-spawning promoter.

The pharmaceutical solution of the present invention can be used to suppress the transient rise in plasma testosterone (in the case of male) concentration (flare phenomenon) observed in administration of a GnRH super-agonist such as leuprorelin acetate. The pharmaceutical solution of the present invention can be used in combination with a GnRH super-agonist such as leuprorelin acetate, gonadorelin, buserelin, triptorelin, goserelin, nafarelin, histrelin, deslorelin, meterelin and lecirelin, with preference given to leuprorelin acetate.

It is also beneficial to use the pharmaceutical solution of the present invention in combination with at least one member selected from the steroidal or nonsteroidal anti-androgen agent or anti-estrogen agent, chemotherapeutic agent, peptide GnRH antagonist, 5α-reductase inhibitor, α-receptor inhibitor, aromatase inhibitor, 17β-hydroxysteroid dehydrogenase inhibitor, adrenal androgen production inhibitor, phosphorylase inhibitor, drugs for hormone therapy, and a pharmaceutical agent inhibiting the action of a growth factor or its receptor, and the like.

Examples of the "chemotherapeutic agent" include ifosfamide, UFT, adriamycin, peplomycin, cisplatin, cyclophosphamide, 5-FU, methotrexate, mitomycin C, mitoxantrone, taxotere, and the like.

Examples of the "peptide GnRH antagonist " include peptide GnRH antagonist for parenteral administration such as cetrorelix, ganirelix, abarelix and the like.

Examples of the "adrenal androgen production inhibitor" include lyase (C_{17,20}-lyase) inhibitors, and the like.

Examples of the "phosphorylase inhibitor" include tyrosine phosphorylase, and the like.

Examples of the "drugs for hormone therapy" include anti-estrogens, progesterones (e.g., MPA, etc.), androgens, estrogens and anti-androgen agent, and the like.

The "growth factor" may be any substance that promotes proliferation of cells and generally includes peptides with molecular weights of not more than 20,000, which expresses the action at low concentrations through binding to receptors. Specifically, there can be mentioned (1) EGF (epidermal growth factor) or substances having substantially the same activity (e.g., EGF, heregulin (HER2 ligand), etc.), (2) insulin or substances having substantially the same activity (e.g., insulin, IGF (insulin-like growth factor)-1, IGF-2, etc.), (3) FGF (fibroblast growth factor) or substances having substantially the same activity (e.g., aFGF, bFGF, KGF (keratinocyte growth factor), HGF (hepatocyte growth factor), FGF-10, etc.), and (4) other growth factors (e.g., CSF (colony stimulating factor), EPO (erythropoietin), IL-2 (interleukin-2), NGF (nerve growth factor), PDGF (platelet-derived growth factor) and TGFβ (transforming growth factor β), etc.), and the like.

The "growth factor receptor" may be any receptor capable of binding the above-mentioned growth factor, including EGF receptor, heregulin receptor (HER2), insulin receptor-1, insulin receptor-2, IGF receptor, FGF receptor-1, FGF receptor-2 and the like.

Examples of the above-mentioned pharmaceutical agent inhibiting the action of the growth factor include herceptin (HER2 receptor antibody) and the like.

Examples of the above-mentioned pharmaceutical agent inhibiting the action of the growth factor or its receptor include herbimycin, PD153035 (Science, 265 (5175) p.1093, (1994)) and the like.

The pharmaceutical agent inhibiting the action of the growth factor or its receptor also includes HER2 inhibitors. The HER2 inhibitors may be any substance that inhibits the activity of HER2 (e.g., phosphorylating activity), thus including an antibody, a low molecular weight compound' (synthetic or natural product), an antisense, a HER2 ligand, heregulin, and any of them as partially modified or mutated in the structure. Moreover, it may be a substance which inhibits HER2 activity by antagonizing a HER2 receptor (e.g. HER2 receptor antibody). Examples 'of the low molecular weight compound having HER2 inhibitory activity include, for example, compounds described in WO 98/03505, namely 1-[3-[4-[2-((E)-2-phenylethenyl)-4-oxazolylmethoxy]phenyl]propyl]-1,2,4-triazole and the like.

For prostatomegaly, combined use of a pharmaceutical agent such as GnRH super-agonist, anti-androgen agent, anti-estrogen agent, peptide GnRH antagonist, 5α-reductase inhibitor, α-receptor inhibitor, aromatase inhibitor, 17β-hydroxysteroid dehydrogenase inhibitor, adrenal androgen production inhibitor, phosphorylase inhibitor and the like, and the pharmaceutical solution of the present invention can be mentioned.

For prostatic cancer, combined use of a pharmaceutical agent such as GnRH super-agonist, anti-androgen agent, anti-estrogen agent, chemotherapeutic agent (e.g., ifosfamide, UFT, adriamycin, peplomycin, cisplatin, etc.), peptide GnRH antagonist, aromatase inhibitor, 17β-hydroxysteroid dehydrogenase inhibitor, adrenal androgen production inhibitor, phosphorylase inhibitor, drugs for hormone therapy such as estrogens (e.g., DSB, EMP, etc.), anti-androgen agent (e.g., CMA etc.) and the like, a pharmaceutical agent inhibiting the action of a growth factor or its receptor, and the like, and the pharmaceutical solution of the present invention can be mentioned.

For breast cancer, combined use of a pharmaceutical agent such as GnRH super-agonist,.anti-estrogen agent, chemotherapeutic agent [e.g., cyclophosphamide, 5-FU, UFT, methotrexate, adriamycin, mitomycin C, mitoxantrone and the like], peptide GnRH antagonist, aromatase inhibitor, adrenal androgen production inhibitor, phosphorylase inhibitor, drugs for hormone therapy [e.g., anti-estrogen agent [e.g., tamoxifen and the like], progesterones (e.g., MPA and the like), androgens, estrogens and the like], a pharmaceutical agent inhibiting the action of a growth factor or its receptor, and the like, and the pharmaceutical solution of the present invention can be mentioned.

The above-mentioned pharmaceutical agent may be administered to the same subject concurrently with the pharmaceutical solution of the present invention, or after some interval. The pharmaceutical solution of the present invention may be administered prior to administration of the GnRH super-agonist such as leuprorelin acetate so as to conduct a treatment while preventing occurrence of flare phenomenon.

While the dose of the pharmaceutical solution of the present invention varies depending on the kind and content of the physiologically active substance, dosage form, the time of sustained release of the physiologically active substance, target disease, target animal and the like, it may be an effective amount of the physiologically active substance. For example, when the pharmaceutical solution is a 24 hour sustained release preparation, the dose of the physiologically active substance per administration is about 0.01-20 mg/kg body weight, preferably about 0.05 to 10 mg/kg body weight, for an adult (body weight 60 kg). When it is a one month preparation, the dose is about 0.01-40 mg/kg body weight, preferably about 0.05 to 10 mg/kg body weight, for an adult (body weight 60 kg).

The dose of the pharmaceutical solution of the present invention per administration is about 0.05 to 50 mg/kg, preferably about 0.1 to 30 mg/kg, for an adult (body weight 60 kg).

The administration frequency is once in several weeks, once in one month, once in several months (e.g., 3 months, 4 months, 6 months and the like), and the like, which may be selected appropriately in consideration of the kind and content of the physiologically active substance, dosage form, the period of sustained release of the physiologically active substance, target disease, target animal and the like.

### Best Mode for Embodying the Invention

### (Examples)

The present invention is explained in more detail in the following by referring to Reference Examples, Examples and Experimental Examples, which are not to be construed as limitative. In the following Reference Examples and Examples, "room temperature" means about 15°C to about 25°C.

### Reference Example 1

### Ethyl 2-amino-4-methyl-5-(4-nitrophenyl)thiophene-3-carboxylate

A mixture of 4-nitrophenylacetone (35.0 g, 195 mmol), ethyl cyanoacetate (23.8 g, 195 mmol), ammonium acetate (3.1 g, 40 mmol) and acetic acid (9.1 ml, 159 mmol) was heated under reflux for 24 hours, with removing water produced with a Dean-Stark trap. After cooling, the reaction mixture was concentrated under reduced pressure and the residue was partitioned between dichloromethane and aqueous sodium hydrogen carbonate solution. The organic layer was washed with aqueous sodium chloride solution and dried (MgSO₄) and the solvent was distilled off under reduced pressure. The residue was purified by silica gel column chromatography to give oil. The oil thus obtained was dissolved in ethanol followed by addition of sulfur (5.0 g, 160 mmol) and diethylamine (16.0 ml, 160 mmol), and the mixture was stirred at 60 to 70°C for 2 hours. After cooling, the reaction mixture was concentrated under reduced pressure to yield residue, which was partitioned between dichloromethane and aqueous sodium hydrogen carbonate solution. The organic layer was washed with aqueous sodium chloride solution and dried (MgSO₄) and the solvent was distilled off under reduced pressure. The residue was purified by silica gel column chromatography to give the crude product, which was crystallized from ether-hexane to give the title compound as red plates (22.2 g, 52%).
mp: 168-170°C (recrystallized from ether-hexane).

| Elemental analysis for C₁₄H₁₄N₂O₄S | | | |
|---|---|---|---|
| | C (%) | H (%) | N (%) |
| Calculated | 54.89 ; | 4.61 ; | 9.14 |
| Found | 54.83 ; | 4.90 ; | 9.09 |

¹H-NMR (200MHz, CDCl₃) δ: 1.39 (3H, t, J=7.1Hz), 2.40 (3H, s), 4.34 (2H, q, J=7.1Hz), 6.27 (2H, br), 7.48 (2H, d, J=8.7Hz), 8.23 (2H, d, J=8.7Hz).
IR (KBr): 3446, 3324, 1667, 1580, 1545, 1506, 1491, 1475, 1410, 1332 cm⁻¹.

### Reference Example 2

### 5-Methyl-6-(4-nitrophenyl)-3-phenylthieno[2,3-d)pyrimidine-2,4 (1H,3H)-dione

To a solution of the compound obtained in Reference Example 1 (5.00 g, 16.32 mmol) in pyridine (30 ml) was added phenyl isocyanate (2.66 ml, 24.48 mmol). After 6 hours of stirring at 45°C, the reaction mixture was concentrated under reduced pressure and the residue thus obtained was dissolved in ethanol (6 ml). To this solution was added 28% sodium methoxide (7.86 g, 40.80 mmol), and the reaction mixture was stirred at room temperature for 2 hours. Then, 2N-hydrochloric acid (25 ml, 50 mmol) was added and the solvent ethanol was distilled off under reduced pressure. The obtained residue was filtered, washed with water-ethanol, dried under reduced pressure, and recrystallized from ethanol to give the title compound as yellow powder (6.09 g, 98%).
mp: >300°C.

| Elemental analysis for C₁₉H₁₃N₃O₄S·0.3H₂O | | | |
|---|---|---|---|
| | C (%) | H (%) | N (%) |
| Calculated | 59.30 ; | 3.56 ; | 10.92 |
| Found | 59.56 ; | 3.52 ; | 10.93 |

¹H-NMR (300MHz, DMSO-d₆) δ: 2.50 (3H, s), 7.31-7.46 (5H, m), 7.78 (2H, d, J=8.8Hz), 8.32 (2H, d, J=8.8Hz), 12.50 (1H, s). IR (KBr) : 1715, 1657, 1593, 1510 cm⁻¹.

### Reference Example 3

### 1-(2,6-Difluorobenzyl)-5-methyl-6-(4-nitrophenyl)-3-phenylthieno[2,3-d]pyrimidine-2,4(1H,3H)-dione

To a solution of the compound obtained in Reference Example 2 (52.54 g, 0.131 mol) in dimethylformamide (1.0 1) were added potassium carbonate (19.00 g, 0.138 mol), potassium iodide (22.90 g, 0.138 mol) and 2,6-difluorobenzyl chloride (22.40 g, 0.138 mol), and the mixture was stirred at room temperature for 2 hours. This reaction mixture was concentrated to give the residue, which was partitioned between chloroform and aqueous sodium chloride solution. The aqueous layer was extracted with chloroform. The combined extracts were washed with aqueous sodium chloride solution and dried (MgSO₄ and the solvent was distilled off under reduced pressure. The residue thus obtained was purified by silica gel column chromatography to give the title compound as light-yellow crystals (61.50 g, 93%).
mp: 280-282°C.

| Elemental analysis for C₂₆H₁₇N₃O₄SF₂ | | | |
|---|---|---|---|
| | C (%) | H (%) | N (%) |
| Calculated | 61.78 ; | 3.39 ; | 8.31 |
| Found | 61.67 ; | 3.46 ; | 8.21 |

¹H-NMR (300MHz, CDCl₃) δ: 2.57 (3H, s), 5.38 (2H, s), 6.94 (2H, d, J=8.1Hz), 7.42-7.58 (8H, m), 8.29 (2H, d, J=8.8Hz).
IR (KBr): 1719, 1669, 1524, 1473 cm⁻¹.

### Reference Example 4

### 5-Bromomethyl-1-(2,6-difluorobenzyl)-6-(4-nitrophenyl)-3-phenylthieno[2,3-d]pyrimidine-2,4(1H,3H)-dione

A mixture of the compound obtained in Reference Example 3 (30.34 g, 0.060 mol), N-bromosuccinimide (12.81 g, 0.072 mol), α,α'-azobisisobutyronitrile (1.15 g, 0.007 mol) and chlorobenzene (450 ml) was stirred at 85°C for 3 hours. After cooling, the reaction mixture was washed with aqueous sodium chloride solution and dried (MgSO₄ and the solvent was then distilled off under reduced pressure. The residue thus obtained was recrystallized from ethyl acetate to give the title compound as yellow needles (80.21 g, 100%).
mp: 228-229°C.
¹H-NMR (300MHz, CDCl₃) δ: 4.77 (2H, s), 5.38 (2H, s), 6.96 (2H, t, J=8.1Hz), 7.29-7.58 (6H, m), 7.79 (2H, d, J=8.5Hz), 8.35 (2H, d, J=8.5Hz).
IR (KBr): 1721, 1680, 1524, 1473, 1348 cm⁻¹.
FAB-Mass m/z 584 (MH)⁺

### Reference Example 5

### 5-(N-Benzyl-N-methylaminomethyl)-1-(2,6-difluorobenzyl)-6-(4-nitrophenyl)-3-phenylthieno[2,3-d]pyrimidine-2,4(1H,3H)-dione

To a solution of the compound obtained in Reference Example 4 (80.00 g, 0.119 mol) in dimethylformamide (600 ml) were added ethyldiisopropylamine (27.00 ml, 0.155 mol) and benzylmethylamine (18.45 ml, 0.143 mol) with ice-cooling.
After 2 hours of stirring at room temperature, the reaction mixture was concentrated and the residue thus obtained was partitioned between ethyl acetate and saturated aqueous sodium hydrogen carbonate solution. The aqueous layer was extracted with ethyl acetate. The organic layers were combined and dried (MgSO₄), and the solvent was distilled off under reduced pressure. The residue thus obtained was purified by silica gel column chromatography to give a yellow oil (74.90 g, 100%), which was recrystallized from ethyl acetate to give the title compound as yellow needles.
mp: 173-174°C.

| Elemental analysis for C₃₄H₂₆N₄O₄SF₂·0.5H₂O | | | |
|---|---|---|---|
| | C(%) | H(%) | N(%) |
| Calculated | 64.45 ; | 4.29 ; | 8.84 |
| Found | 64.50 ; | 4.24 ; | 8.82 |

¹H-NMR (300MHz, CDCl₃) [Free amine] δ: 1.31 (3H, s), 3.60 (2H, s), 3.96 (2H, s), 5.39 (2H, s), 6.95 (2H, t, J=8.2Hz), 7.18-7.55 (11H, m), 8.02 (2H, d, J=9.0Hz), 8.26 (2H, d, J=9.0Hz).
IR (KBr) [hydrochloride]: 1719, 1678, 1597, 1520 cm⁻¹.

### Reference Example 6

### 6-(4-Aminophenyl)-5-(N-benzyl-N-methylaminomethyl)-1-(2,6-difluorobenzyl)-3-phenylthieno[2,3-d]pyrimidine-2,4(1H,3H)-dione

To a solution of the compound obtained in Reference Example 5 (3.00 g, 4.80 mmol) in formic acid (30 ml) were added 1M hydrogen chloride - ether (14.4 ml, 14.4 mmol) and 10% palladium-on-carbon powder (300 mg) with ice-cooling, and hydrogenation was carried out under atmospheric condition at room temperature with stirring for 2 hours. This reaction mixture was filtered through Celite and the filtrate was concentrated under reduced pressure. The residue thus obtained was partitioned between dichloromethane and saturated aqueous sodium hydrogen carbonate solution. The aqueous layer was extracted with dichloromethane and the organic layers were combined and dried (MgSO₄). The solvent was then distilled off under reduced pressure. The residue thus obtained was purified by silica gel column chromatography to give the title compound as white crystals (2.41 g, 84%).
mp: 205-207°C.

| Elemental analysis for C₃₄H₂₈N₄O₂SF₂·0.1AcOEt·1.2H₂O | | | |
|---|---|---|---|
| | C(%) | H(%) | N(%) |
| Calculated | 66.09 ; | 5.03; | 8.96 |
| Found | 66.93 ; | 4.94 ; | 8.67 |

¹H-NMR (300MHz, CDCl₃) δ: 2.05 (3H, s), 3. 56 (2H, s), 3. 83 (2H, br), 3.88 (2H, s), 5.36 (2H, s), 6.70 (2H, d, J=8.8Hz), 6.88-6.94 (2H, m), 7.21-7.31 (8H, m), 7.41-7.53 (5H, m).
IR (KBr) : 1715, 1657, 1628, 1537 cm⁻¹.

### Reference Example 7

### 5-(N-Benzyl-N-methylaminomethyl)-1-(2,6-difluorobenzyl)-6-[4-(3-methoxyureido)phenyl]-3-phenylthieno[2,3-d]pyrimidine-2,4(1H,3H)-dione.

To a solution of the compound (5.0 g, 8.41 mmol) obtained in Reference Example 6 in dichloromethane (120 ml) was added triethylamine (2.34 ml, 16.82 mmol) under ice-cooling, and the mixture was stirred. To the reaction mixture was added N,N'-carbonyldiimidazole (2.73 g, 16.82 mmol) under ice-cooling, and the mixture was allowed to warm from under ice-cooling to room temperature. The mixture was stirred for 42 hours. The mixture was cooled to under ice-cooling again, and O-methylhydroxylamine hydrochloride (7.02 g, 84.08 mmol) and triethylamine (11.7 ml, 84.08 mmol) were added. The reaction mixture was allowed to warm from under ice-cooling to room temperature, and stirred for 3 hours. The reaction mixture was partitioned between chloroform and saturated aqueous sodium hydrogen carbonate solution. The aqueous layer was extracted with chloroform, the extracts were combined, washed with brine and dried over MgSO₄, after which the solvent was evaporated under reduced pressure. The obtained residue was purified by silica gel column chromatography to give a pale-yellow solid, which was recrystallized from chloroform-ether to give the title compound as white crystals (4.52 g, 80%.
mp: 204-205°C.

| Elemental analysis for C₃₆H₃₁N₅O₄SF₂ | | | |
|---|---|---|---|
| | C (%) | H (%) | N (%) |
| Calculated | 64.75; | 4.68; | 10.49 |
| Found | 64.61; | 4.67; | 10.31 |

¹H-NMR (300MHz, CDCl₃) δ: 2.05 (3H, s), 3.57 (2H, s), 3.82 (3H, s), 3.90 (2H, s), 5.37 (2H, s), 6.92 (2H, d, J=8.2Hz), 7.16-7.31 (9H, m), 7.42-7.57 (5H, m), 7.63 (1H, s), 7.73 (2H, d, J=8.8Hz).
IR(KBr): 3338, 3064, 1717, 1669, 1628, 1591, 1531, 1470cm⁻¹.

### Reference Example 8

### 3-(N-Benzyl-N-methylaminomethyl)-4,7-dihydro-5-isobutyryl-7-(2,6-difluorobenzyl)-2-[4-[(1-hydroxycyclopropyl)-carbonylamino]phenyl]-4-oxothieno[2,3-b]pyridine

To a solution of 2-(4-aminophenyl)-7-(2,6-difluorobenzyl)-4,7-dihydro-5-isobutyryl-3-(N-benzyl-N-methylaminomethyl)-4-oxothieno[2,3-b]pyridine (0.57 g, 1.0 mmol) in dichloromethane (10 ml) were added diisopropylethylamine (0.52 g, 4 mmol) and 1-hydroxycyclopropanecarboxylic acid (0.204 g, 2 mmol), and the mixture was stirred under ice-cooling. To this solution was added benzotriazol-1-yloxytrisdimethylaminophosphonium hexafluorophosphate (BOP reagent) (1.76 g, 4 mmol). The mixture was stirred under ice-cooling for 1 hour and further at room temperature for 4 days. The reaction mixture was concentrated to dryness under reduced pressure, and the obtained residue was partitioned between water (50 ml) and chloroform (50 ml). The aqueous layer was again extracted with chloroform (10 ml), the extracts were combined, washed with brine and dried over MgSO₄, after which the solvent was evaporated under reduced pressure. The obtained residue was purified by silica gel column chromatography and recrystallized from ether to give yellow powder crystals (0.27 g, 41%).
¹H-NMR (300MHz, CDCl₃) δ: 1.16-1.20 (2H, m), 1.18 (6H, d), 1.48-1.51 (2H, m), 2.09 (3H, s), 3.64 (2H, s), 3.95 (1H, br s), 4.14 (2H, s), 4.12-4.19 (1H, m), 5.20 (2H, s), 6.99 (2H, t), 7.10-7.25 (5H, m), 7.34-7.46 (1H, m), 7.57 (2H, d), 7.70 (2H, d), 8.21 (1H, s), 8.82 (1H, s).

### Reference Example 9

Compound (XIII) was synthesized according to the method described in Proceedings of the National Academy of Sciences (1998), vol. 95, pp. 10836-10841.

### Example 1

The compound (200 mg) obtained in Reference Example 7 and salicylic acid (62.1 mg) were added to dimethyl sulfoxide (2 mL) and, after mixing with a vortex mixer, the mixture stood overnight at room temperature to allow dissolution.

### Example 2

The compound (1200 mg) obtained in Reference Example 7 and salicylic acid (372.6 mg) were added to dimethyl sulfoxide (2 mL) and, after mixing with a vortex mixer, the mixture stood overnight at room temperature to allow dissolution.

### Example 3

The compound (200 mg) obtained in Reference Example 8 and salicylic acid (42.1 mg) were added to dimethyl sulfoxide (2 mL) and, after mixing with a vortex mixer, the mixture stood overnight at room temperature to allow dissolution.

### Example 4

The compound (XIII) (25 mg) synthesized in Reference Example 9 and 3-hydroxy-2-naphthoic acid (14.6 mg) were added to polyethylene glycol 300 (2.0 mL) and, after mixing with a vortex mixer, the mixture stood overnight at room temperature to allow dissolution.

### Example 5

The compound (XIII) (75 mg) synthesized in Reference Example 9 and 3-hydroxy-2-naphthoic acid (43.7 mg) were added to polyethylene glycol 300 (1.9 mL) and, after mixing with a vortex mixer, the mixture stood overnight at room temperature to allow dissolution.

### Example 6

The solution (200 µL) prepared in Example 2 was charged in a subcutaneous implantation type minipump (model 2004, manufactured by Alza Corporation).

### Example 7

The solution (200 µL) prepared in Example 5 was charged in a subcutaneous implantation type minipump (model 2001, manufactured by Alza Corporation).

### Experimental Example 1

The subcutaneous implantation type minipump prepared in Example 6 was subcutaneously implanted in the back of 7-week-old male SD rats. After a predetermined time after the implantation, blood was drawn from the tail vein of the rats, and serum was separated. The shift of the drug concentration of the obtained serum with time is shown in Table 1 (average value: n=4).

**Table 1**

| Time (days) after administration | Concentration in serum (ng/mL) |
|---|---|
| 0 | 0.0 |
| 2 | 6.0 |
| 4 | 8.4 |
| 7 | 19.9 |
| 9 | 10.1 |
| 11 | 24.1 |
| 14 | 12.0 |
| 16 | 25.3 |
| 18 | 0.6 |
| 21 | 9.0 |

The results of Table 1 have confirmed that the compound obtained in Reference Example 7 was released in a sustained manner for 21 days.

### Experimental Example 2

The subcutaneous implantation type minipump prepared in Example 7 was subcutaneously implanted in the back of 26-week-old male Wistar fatty rats. After a predetermined time after the implantation, blood was drawn from the tail vein of the rats, and serum was separated. The shift of the drug concentration of the obtained serum with time is shown in Table 2 (average value: n=5).

**Table 2**

| Time (days) after administration | Concentration in serum (ng/mL) |
|---|---|
| 0 | 0.0 |
| 1 | 43.7 |
| 3 | 60.0 |
| 5 | 94.8 |
| 7 | 37.1 |

The results of Table 2 have revealed that the concentration of compound (XIII) in serum was maintained high for almost 7 days by the use of the one week type pump.

### Industrial Applicability

The pharmaceutical solution of the present invention comprises a physiologically active non-peptide substance, an organic acid and a biocompatible organic solvent. This pharmaceutical solution can contain a physiologically active non-peptide substance at a concentration higher than the solubility of the physiologically active non-peptide substance in a biocompatible organic solvent. As a result, the content of the physiologically active non-peptide substance in the preparation can be made higher. This makes it possible to afford a preparation having a size acceptable for administration to organisms. In addition, downsizing of preparation can reduce burden on patients.

This application is based on a patent application No. 099578/2001 filed in Japan, the contents of which are all hereby incorporated by reference.

## Claims

1. A pharmaceutical solution comprising a physiologically active non-peptide substance, an organic acid and a biocompatible organic solvent.

2. The pharmaceutical solution of claim 1, wherein the organic acid is one or more kinds selected from a lower fatty acid, an aliphatic hydroxycarboxylic acid and an aromatic organic acid.

3. The pharmaceutical solution of claim 2, wherein the aliphatic hydroxycarboxylic acid is lactic acid.

4. The pharmaceutical solution of claim 2, wherein the aromatic organic acid is an aromatic hydroxycarboxylic acid.

5. The pharmaceutical solution of claim 4, wherein the aromatic hydroxycarboxylic acid is salicylic acid.

6. The pharmaceutical solution of claim 4, wherein the aromatic hydroxycarboxylic acid is 1-hydroxy-2-naphthoic acid or 3-hydroxy-2-naphthoic acid.

7. The pharmaceutical solution of claim 4, wherein the aromatic hydroxycarboxylic acid is pamoic acid.

8. The pharmaceutical solution of claim 4, wherein the aromatic hydroxycarboxylic acid is a mixture of two or more kinds selected from salicylic acid, 1-hydroxy-2-naphthoic acid, 3-hydroxy-2-naphthoic acid and pamoic acid.

9. The pharmaceutical solution of claim 1, wherein the physiologically active non-peptide substance is a basic substance.

10. The pharmaceutical solution of claim 1, wherein the physiologically active non-peptide substance has a molecular weight of not more than about 1,000.

11. The pharmaceutical solution of claim 1, wherein the biocompatible organic solvent is polyethylene glycol or a fatty acid ester thereof, or dimethyl sulfoxide.

12. The pharmaceutical solution of claim 1, which further comprises a hydrophilic polymer.

13. The pharmaceutical solution of claim 1, wherein the content of the physiologically active non-peptide substance is not less than about 5 wt% of the total weight of the solution.

14. The pharmaceutical solution of claim 1, wherein the content of the organic acid is about 1 wt% to about 40 wt% of the total weight of the solution.

15. The pharmaceutical solution of claim 1, wherein the organic acid is contained in a proportion of about 1/20 to about 100 moles per 1 mole of the physiologically active non-peptide substance.

16. The pharmaceutical solution of claim 1, wherein the physiologically active non-peptide substance is contained at a concentration higher than the solubility of the physiologically active non-peptide substance in the biocompatible organic solvent.

17. The pharmaceutical solution of claim 1, which is a preparation for parenteral administration.

18. The pharmaceutical solution of claim 17, which is a preparation for injection.

19. A preparation for implantation, which comprises the pharmaceutical solution of claim 1.

20. The pharmaceutical solution of claim 1, which is a preparation for oral administration.

21. A capsule comprising the pharmaceutical solution of claim 1.
